# NEW EUROPEAN PATENT SPECIFICATION

(11) **EP 0 640 619 B2**
(45) Date of publication and mention of the opposition decision: **23.03.2005**
(45) Mention of the grant of the patent: 23.07.1997
(21) Application number: 94112732.6
(22) Date of filing: 16.08.1994
(51) Int. Cl.: C07K 14/505, A61K 38/18, C12N 15/16

(54) **Erythropoietin analogs with additional glycosylation sites**
Erythropoietin-Analoga mit zusätzlichen Glycosylierungsstellen
Analogues d'érythropoiétine avec des sites additionnels de glycosylation

(30) Priority: 17.08.1993 US 108016
(43) Date of publication of application: 01.03.1995
(73) Proprietor: Kirin-Amgen, Inc., Thousand Oaks, CA 91320-1799 (US)
(72) Inventor: Elliot, Steven G., Newbury Park, California 91320 (US); Byrne, Thomas E., Arlington, Virginia 22209 (US)
(74) Representative: Vossius, Volker, Dr.

(56) References cited:
- EP-A- 0 267 678
- EP-A- 0 428 267

### SEQUENCE LISTING

(1) GENERAL INFORMATION:
   (i) APPLICANT: Amgen Inc.
   (ii) TITLE OF INVENTION: Erythropoietin Analogs
   (iii) NUMBER OF SEQUENCES: 26
   (iv) CORRESPONDENCE ADDRESS:
      (A) ADDRESSEE: Amgen Inc.
      (B) STREET: Amgen Center, 1840 DeHavilland Drive
      (C) CITY: Thousand Oaks
      (D) STATE: California
      (E) COUNTRY: U.S.A.
      (F) ZIP: 91320-1789
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
(2) INFORMATION FOR SEQ ID NO:1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:1:
   CGCCCACCAA ACCTCAGCTG TGACAGCCGA 30
(2) INFORMATION FOR SEQ ID NO:2:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:2:
   ATCTGTACAA CCGAAGCCTG GAGAGGT 27
(2) INFORMATION FOR SEQ ID NO:3:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
   GGGCCTGGCC AACCTGTCGG AAG 23
(2) INFORMATION FOR SEQ ID NO:4:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   TCCCCTCCAG ATAATGCCTC AGCTGC 26
(2) INFORMATION FOR SEQ ID NO:5:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
   CAGATGCGAA CTCATCTGCT CCAC 24
(2) INFORMATION FOR SEQ ID NO:6:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7:
(2) INFORMATION FOR SEQ ID NO:8:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:8:
   CCTCCAGATC CGACCTCAGC TGC 23
(2) INFORMATION FOR SEQ ID NO:9:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   GGGCCTGGCC AACCTGACAG AAGCTGTC 28
(2) INFORMATION FOR SEQ ID NO:10:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:10:
   CAGGGCCTGT CCAACCTGAC AGAAGCTGTC 30
(2) INFORMATION FOR SEQ ID NO:11:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
   CAGATGCGAA CTCAACGGCT CCAC 24
(2) INFORMATION FOR SEQ ID NO:12:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
   ATGCGAACTC AACGGCTCCA CTCACAACAA TCACT 35
(2) INFORMATION FOR SEQ ID NO:13:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
   CCAGATCCAA ATTCATCTGC TCCACTC 27
(2) INFORMATION FOR SEQ ID NO:14:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
   CCAGATCCAA ATTCAACAGC TCCACTC 27
(2) INFORMATION FOR SEQ ID NO:15:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   CCAGATGCGA CAACAGCTGC TCCA 24
(2) INFORMATION FOR SEQ ID NO:16:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
   AGATCCGACC ACCGCTGCTC CAC 23
(2) INFORMATION FOR SEQ ID NO:17:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
   TGCTCCACTC ACAACAATCA CTG 23
(2) INFORMATION FOR SEQ ID NO:18:
   (i) SEQUENCE CHARACTERISTICS:
      (A) LENGTH: 184 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
   TCGAGGAACT GAAAAACCAG AAAGTTAACT GGTAAGTTTA GTCTTTTTGT CTTTTATTTC     60
   AGGTCCCGGA TCCGGTGGTG GTGCAAATCA AAGAACTGCT CCTCAGTGGA TGTTGCCTTT    120
   ACTTCTAGGC CTGTACGGAA GTGTTACTTC TGCTCTAAAA GCTGCTGCAA CAAGCTGGTC    180
   GACC                                                                 184
(2) INFORMATION FOR SEQ ID NO:19:
   (i) SEQUENCE CHARACTERISTICS:
      (A) LENGTH: 107 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:19:
   CCTGTAGGAC AGGGGACAGA TCCTCTTCCT CAAAGGCCCC TCCCCCCAGC CTTCCAAGTC     60
   CATCCCGACT CCCGGGGCCC TCGGACACCC CGATCCTCCC ACAATGA                  107
(2) INFORMATION FOR SEQ ID NO:20:
   (i) SEQUENCE CHARACTERISTICS:
      (A) LENGTH: 108 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:20:
   GATCTCATTG TGGGAGGATC GGGGTGTCCG AGGGCCCCGG GAGTCGGGAT GGACTTGGAA     60
   GGCTGGGGGG AGGGGCCGAG GAAGAGGATC TGTCCCCTGT CCTACAGG                 108
(2) INFORMATION FOR SEQ ID NO:21:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
   GGAGGCCGAG CAGATCACGA CGG 23
(2) INFORMATION FOR SEQ ID NO:22:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
   CTTGAATGAG CAGATCACTG TCC 23
(2) INFORMATION FOR SEQ ID NO:23:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
   CTGTTGGTCC AGTCTTCCCA G 21
(2) INFORMATION FOR SEQ ID NO:24:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
(2) INFORMATION FOR SEQ ID NO:25:
   (i) SEQUENCE CHARACTERISTICS:
      (A) LENGTH: 28 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:25:
   Ser Ser Ser Ser Lys Ala Pro Pro Pro Ser Leu Pro Ser Pro Ser Arg
   1               5                   10                  15
   Leu Pro Gly Pro Ser Asp Thr Pro Ile Leu Pro Gln
               20                  25
(2) INFORMATION FOR SEQ ID NO:26:
   (i) SEQUENCE CHARACTERISTICS:
      (A) LENGTH: 193 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:26:
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ala Pro Pro Arg Leu
               20                  25                  30
   Ile Cys Asp Ser Arg Val Leu Glu Arg Tyr Leu Leu Glu Ala Lys Glu
           35                  40                  45
   Ala Glu Asn Ile Thr Thr Gly Cys Ala Glu His Cys Ser Leu Asn Glu
       50                  55                  60
   Asn Ile Thr Val Pro Asp Thr Lys Val Asn Phe Tyr Ala Trp Lys Arg
   65                  70                  75                  80
   Met Glu Val Gly Gln Gln Ala Val Glu Val Trp Gln Gly Leu Ala Leu
                   85                  90                  95
   Leu Ser Glu Ala Val Leu Arg Gly Gln Ala Leu Leu Val Asn Ser Ser
               100                 105                 110
   Gln Pro Trp Glu Pro Leu Gln Leu His Val Asp Lys Ala Val Ser Gly
           115                 120                 125
   Leu Arg Ser Leu Thr Thr Leu Leu Arg Ala Leu Gly Ala Gln Lys Glu
       130                 135                 140
   Ala Ile Ser Pro Pro Asp Ala Ala Ser Ala Ala Pro Leu Arg Thr Ile
   145                 150                 155                 160
   Thr Ala Asp Thr Phe Arg Lys Leu Phe Arg Val Tyr Ser Asn Phe Leu
                   165                 170                 175
   Arg Gly Lys Leu Lys Leu Tyr Thr Gly Glu Ala Cys Arg Thr Gly Asp
               180                 185                 190
   Arg

## Description

The present invention relates to erythropoietin analogs having at least one additional site for glycosylation or erythropoietin analogs having a deletion of one or more glycosylation sites in human erythropoietin and an addition of an equal number of non-naturally occuring glycosylation sites. The invention also relates to DNA sequences encoding said erythropoietin analogs, and recombinant plasmids and host cells for analog expression.

### Background of the Invention

Erythropoietin (EPO) is a glycoprotein hormone involved in the maturation or erythroid progenitor cells into erythrocytes. It is essential in regulating levels or red blood cells in circulation. Naturally occurring erythropoietin is produced by the liver during fetal life and by the kidney of adults and circulates in the blood and stimulates the production of red blood cells in bone marrow. Anemia is almost invariably a consequence of renal failure due to decreased production of erythropoietin from the kidney. Recombinant erythropoietin produced by genetic engineering techniques involving the expression of a protein product from a host cell transformed with the gene encoding erythropoietin has been found to be effective when used in the treatment of anemia resulting from chronic renal failure.

Low levels of erythropoietin are normally present in human urine, while individuals suffering from aplastic anemia exhibit elevated levels of urinary erythropoietin. The purification of human urinary erythropoietin by Miyake et al. in J. Biol. Chem., 252, 5558 (1977), used, as starting material, urine from aplastic anemic individuals. To date, however, urinary erythropoietin has not been shown to be therapeutically useful.

The identification, cloning, and expression of genes encoding erythropoietin are described in U. S. Patent No. 4,703,008 to Lin, the disclosure of which is incorporated herein by reference. A description of a method for purification of recombinant erythropoietin from cell medium is included in U. S. Patent No. 4,667,016 to Lai et al. The expression and recovery of biologically active recombinant erythropoietin from mammalian cell hosts containing the erythropoietin gene on recombinant plasmids has, for the first time, made available quantities of erythropoietin suitable for therapeutic applications. In addition, knowledge of the gene sequence and the availability of larger quantities of purified protein has led to a better understanding of the mode of action of this protein.

Many cell surface and secretory proteins produced by eucaryotic cells are modified with one or more oligosaccharide groups. This modification, referred to as glycosylation, can dramatically affect the physical properties of proteins and can also be important in protein stability, secretion, and subcellular localization. Proper glycosylation can be essential for biological activity. In fact, some genes from eucaryotic organisms, when expressed in bacteria (e.g., E. coli) which lack cellular processes for glycosylating proteins, yield proteins that are recovered with little or no activity by virtue of their lack of glycosylation.

EP-A-428267 discloses analogs of human erythropoietin with one or more changes in the amino acid sequence of human erythropoietin which result in an increase in the number of sites for sialic acid attachment. The analogs have a greater number of carbohydrate chains than human erythropoietin.

Glycosylation occurs at specific locations along the polypeptide backbone and is usually of two types: O-linked oligosaccharides are attached to serine or threonine residues while N-linked oligosaccharides are attached to asparagine residues when they are part of the sequence Asn-X-Ser/Thr, where X can be any amino acid except proline. The structures of N-linked and O-linked oligosaccharides and the sugar residues found in each type are different. One type of sugar that is commonly found on both is N-acetylneuraminic acid (hereafter referred to as sialic acid). Sialic acid is usually the terminal residue of both N-linked and O-linked oligosaccharides and, by virtue of its negative charge, may confer acidic properties to the glycoprotein.

Both human urinary derived erythropoietin and recombinant erythropoietin (expressed in mammalian cells) having the amino acid sequence 1-165 of human erythropoietin contain three N-linked and one O-linked oligosaccharide chains which together comprise about 40% of the total molecular weight of the glycoproteln. N-linked glycosylation occurs at asparagine residues located at positions 24, 38 and 83 while O-linked glycosylation occurs at a serine residue located at position 126 (Lai et al. J. Biol. Chem. 261, 3116 (1986); Broudy et al. Arch. Biochem. Biophys. 265, 329 (1988)). The oligosaccharide chains have been shown to be modified with terminal sialic acid residues. Enzymatic treatment of glycosylated erythropoietin to remove all sialic acid residues results in a loss of in vivo activity but does not affect in vitro activity (Lowy et al. Nature 185, 102 (1960); Goldwasser et al. J. Biol. Chem. 249, 4202 (1974)). This behavior has been explained by rapid clearance of asialoerythropoietin from circulation upon interaction with the hepatic asialoglycoprotein binding protein (Morrell et al. J. Biol. Chem. 243, 155 (1968); Briggs, et al. Am. J. Physiol. 227, 1385 (1974); Ashwell et al. Methods Enzymol. 50, 287 (1978)). Thus, erythropoietin possesses in vivo biological activity only when it is sialylated to avoid its binding by the hepatic binding protein.

The role of the other components in the oligosaccharide chains of erythropoietin is not well defined. It has been shown that partially deglycosylated erythropoietin has greatly reduced in vivo activity compared to the glycosylated form but does retain in vitro activity (Dordal et al. Endocrinology 116, 2293 (1985); Lin patent, supra). In another study, however, the removal of N-linked or O-linked oligosaccharide chains singly or together by mutagenesis of asparagine or serine residues that are glycosylation sites sharply reduces in vitro activity of the altered erythropoietin that is produced in mammalian cells (Dube et al. J. Biol. Chem. 263, 17516 (1988)).

Glycoproteins such as erythropoietin can be separated into different charged forms using techniques such as isoelectric focusing (IEF). Several parties have reported IEF studies of crude and partially purified erythropoietin preparations (Lukowsky et al., J. Biochem 50, 909 (1972); Shelton et al. Biochem. Med. 12, 45 (1975); Fuhr et al. Biochem. Biophys. Res. Comm. 98, 930 (1981)). At most, three or four fractions having erythropoietin activity were distinguished by IEF in these studies and none were characterized with respect to carbohydrate content. In addition, no correlation between the isoelectric points of the fractions and their biological activity was made.

During the purification of urinary erythropoietin from human urine discussed in Miyake et. al. supra, two erythropoietin fractions from hydroxylapatite chromatography designated II and IIIA were reported to have similar specific activity. A subsequent carbohydrate analysis of fractions II and IIIA revealed that fraction II had a greater average sialic acid content than fraction IIIA (Dordal et. al. supra).

It is an object of the present invention to provide separated and isolated isoforms of erythropoietin having a defined sialic acid content and increased biological activity. Pharmaceutical compositions containing such molecules would have therapeutic benefit.

### Summary of the Invention

The subject invention relates to analogs of human erythropoietin comprising an amino acid sequence which includes at least one additional site for glycosylation. The added sites for glycosylation may result in a greater number of carbohydrate chains, and higher sialic acid content, than human erythropoietin. Erythropoietin analogs comprising amino acid sequences which include a deletion of one or more glycosylation sites and an addition of an equal number of non-naturally occuring glycosylation sites are also provided. Analogs comprising an addition of one or more amino acids to the carboxy terminal end of erythropoietin wherein the addition provides at least one glycosylation site are also included. The invention further encompasses DNA sequences encoding such erythropoietin analogs, and recombinant plasmids and host cells for analog expression.

### Brief Description of the Drawings

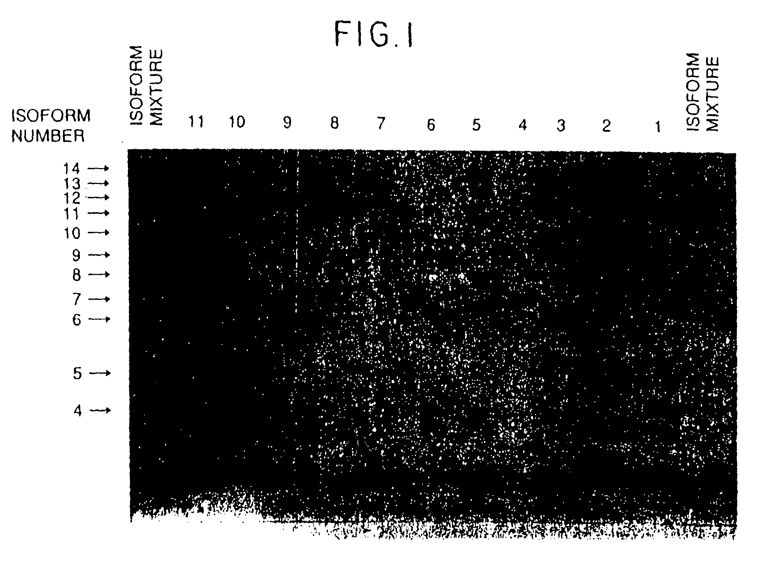

Figure 1 shows an analytical isoelectric focusing gel of the separate recombinant erythropoietin isoforms. Gel lanes 1-11 show isoforms ranging from less acidic (higher pl) in lane 1 to more acidic (lower pl), in lane 11. Purified recombinant erythropoietin containing a mixture of isoforms 9-14 is also shown in the far left and right lanes of the gel.

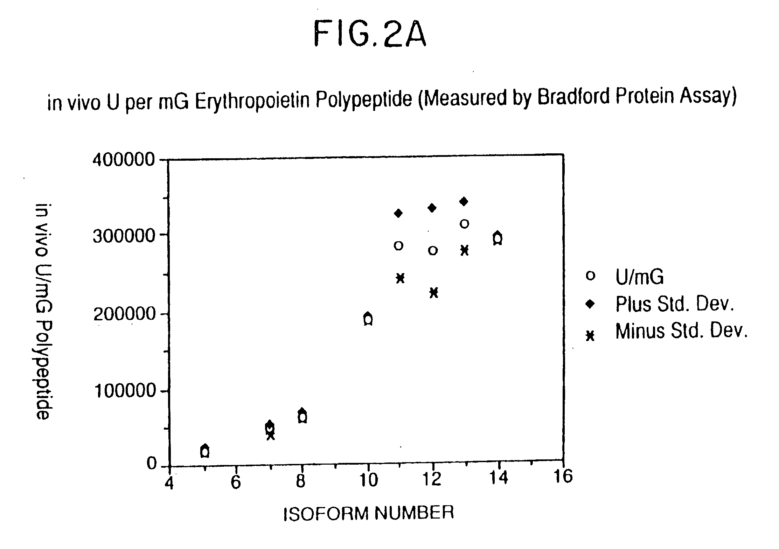

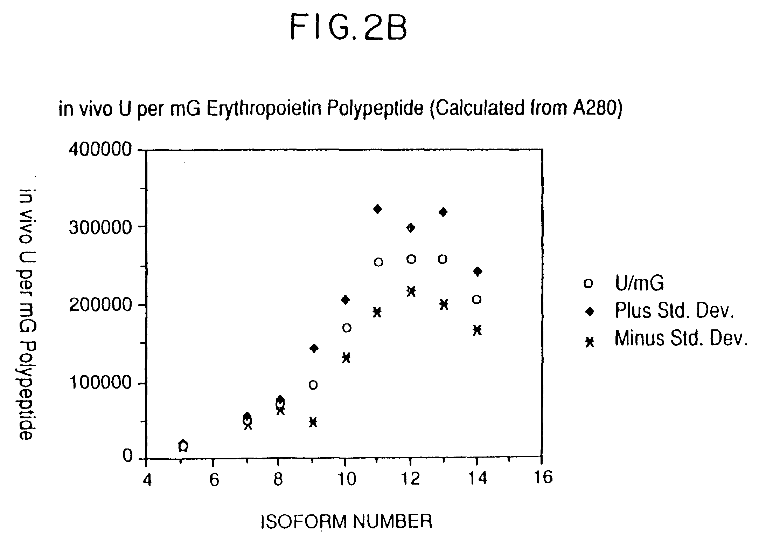

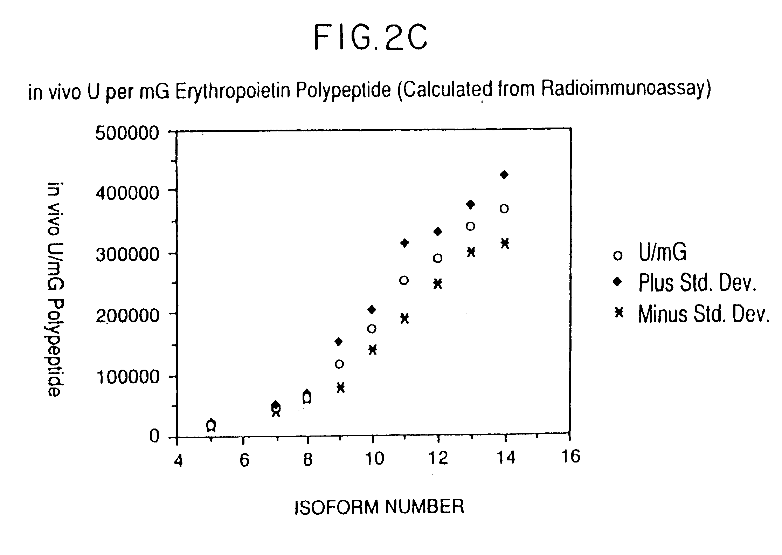

Figure 2 shows the relationship between the number of sialic acids per erythropoietin isoform and the in vivo specific activity of each isoform expressed as units per mg of erythropoietin polypeptide. In Figure 2A, the concentration of each erythropoietin isoform was determined by the Bradford protein assay; in 2B, the concentration was determined by absorbance at 280 nm, in 2C, the concentration was determined by RIA.

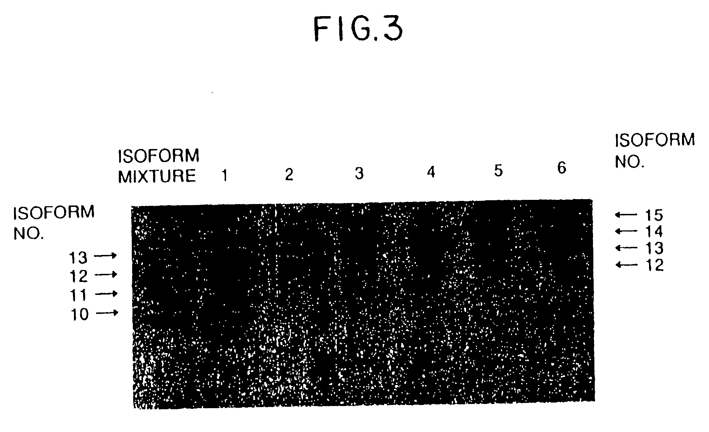

Figure 3 shows an analytical isoelectric focusing gel of defined mixtures of recombinant erythropoietin isoforms prepared by anion exchange chromatography under different conditions. Gel lanes 1-6 represent, respectively, erythropoietin isoforms eluted in a high salt wash after washing the Q-Sepharose fast flow column with 150 mM acetic acid, pH 4.7, 150 mM acetic acid (unbuffered), 200 mM acetic acid, pH 4.7, 250 mM acetic acid, pH 4.7, 300 mM acetic acid, pH 4.7 or 300 mM acetic acid (unbuffered). Purified recombinant erythropoietin containing a mixture of isoforms as obtained using procedures described in Example 2 of Lai et al., supra, except that DEAE-Agarose chromatography is replaced by Q-Sepharose chromatography, is also shown in the far left lane of the gel.

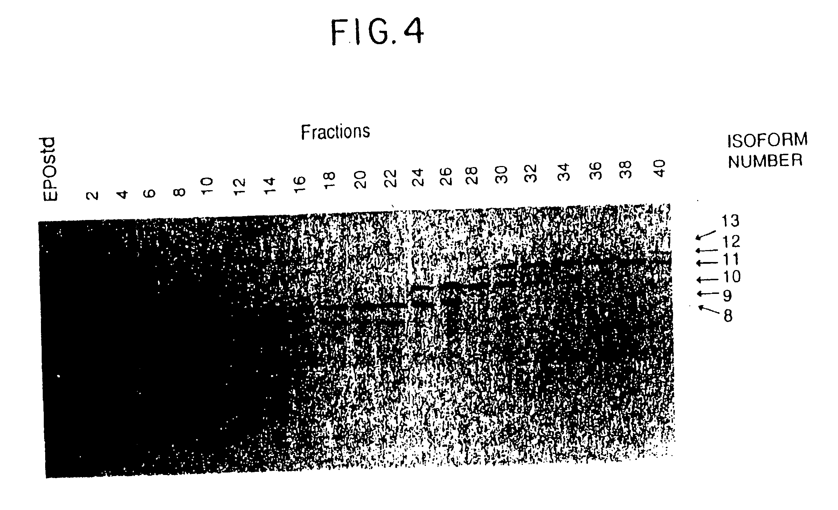

Figure 4 shows the separation of erythropoietin isoforms 8 to 12 achieved by subjecting cell conditioned medium applied to a column of Q-Sepharose to a gradient of decreasing pH and increasing ionic strength. Aliquots of even numbered fractions from Fraction 2 to Fraction 40 were subjected to analytical isoelectric focusing. Purified recombinant erythropoietin containing a mixture of isoforms obtained using procedures described in Example 2 of Lai et al. supra, except that DEAE-Agarose chromatography is replaced by Q-Sepharose chromatography, is also shown in the far left lane of the gel.

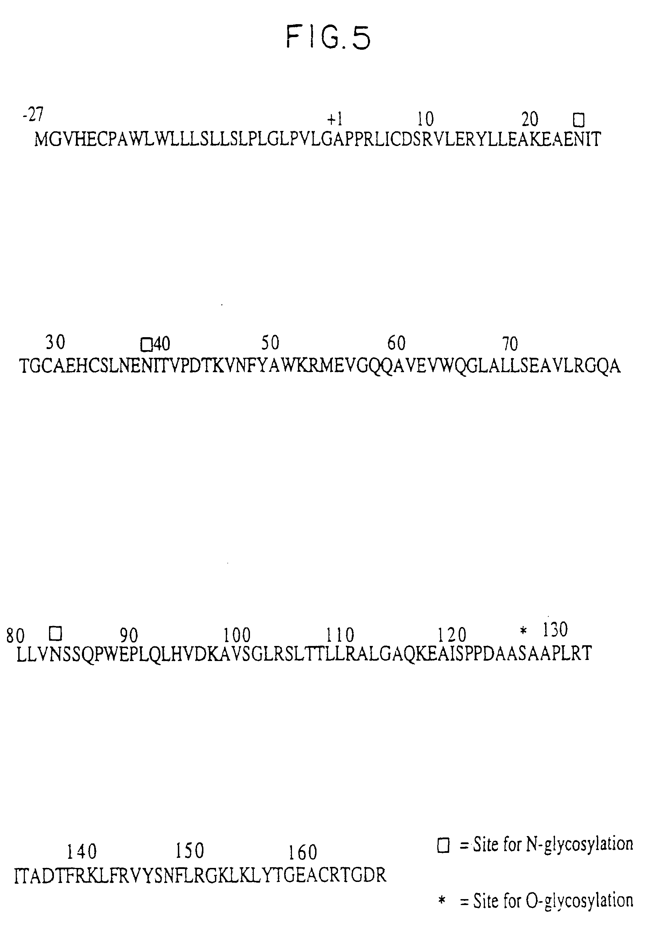

Figure 5 shows the amino acid sequence of human erythropoietin. Squares indicate asparagine residues to which N-linked carbohydrate chains are attached and an asterisk indicates the serine residue modified with an O-linked carbohydrate chain.

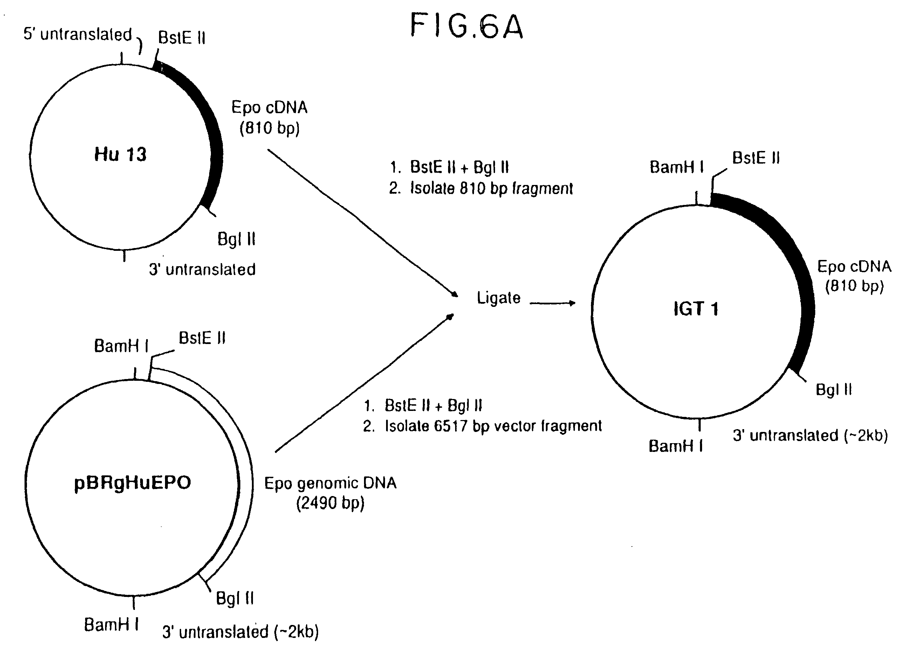

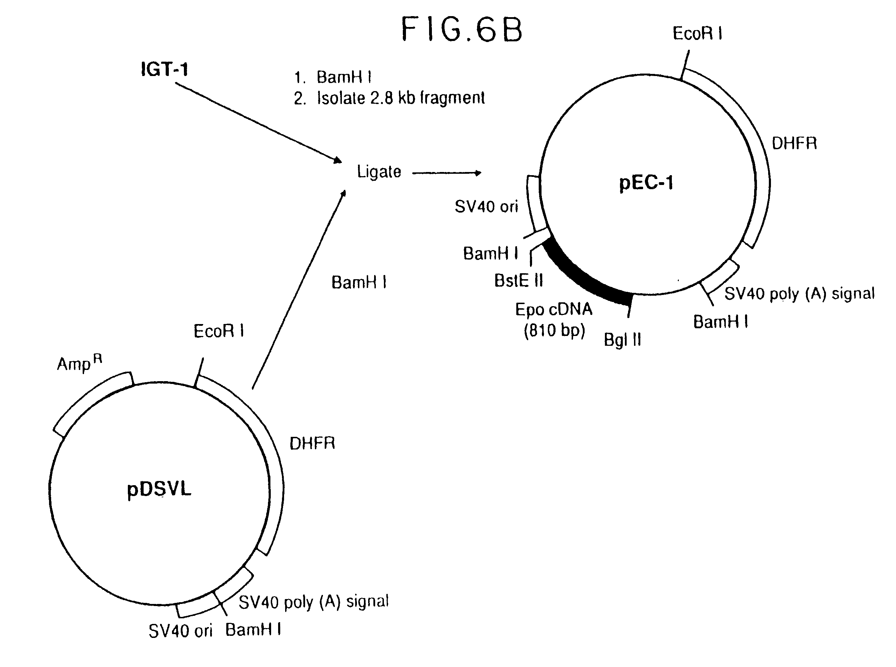

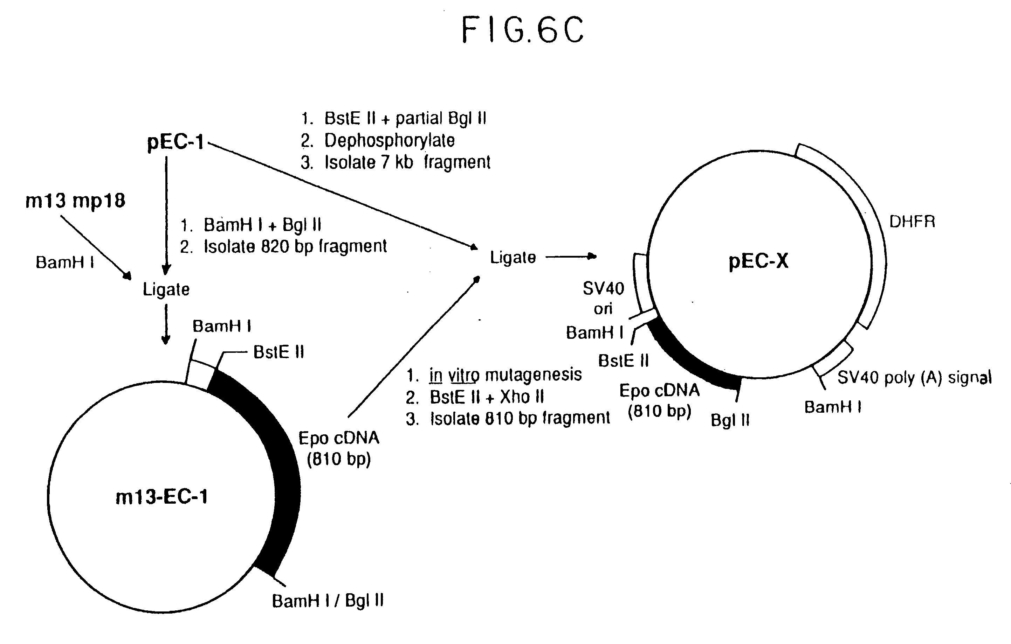

Figures 6A, 6B, and 6C show the series of cloning steps used in generating plasmids for the construction and analysis of analogs of human erythropoietin. These analogs have amino acids altered as shown in Figure 5 which provide additional glycosylation sites.

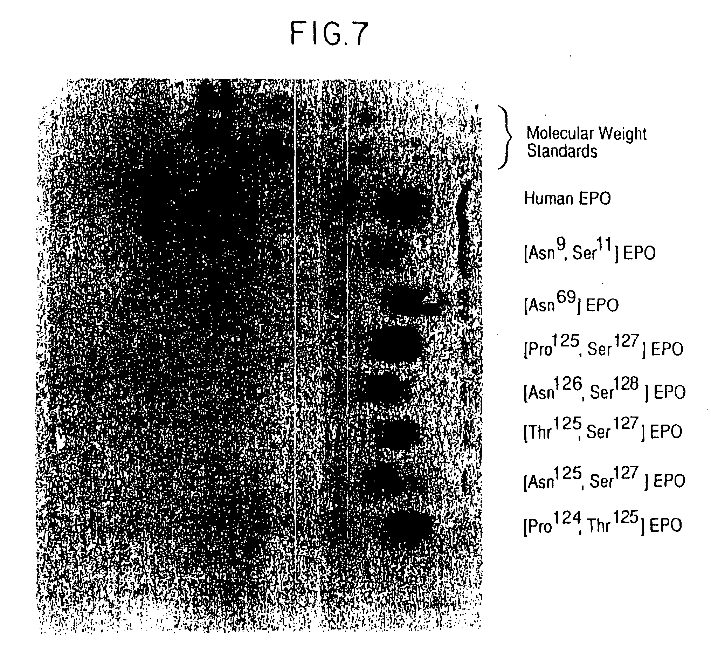

Figure 7 shows a Western blot analysis of COS cell supernatants of human sequence erythropoietin and indicated erythropoietin analogs. The analogs [Asn⁹, Ser¹¹] EPO, [Asn⁶⁹] EPO, [Asn¹²⁵, Ser¹²⁷] EPO, and [Pro¹²⁴, Thr¹²⁵] EPO are constructed as described in Example 6. Additional analogs [Pro¹²⁵, Thr¹²⁷] EPO and [Asn¹²⁶, Ser¹²⁸] EPO which do not contain additional carbohydrate chains are shown for comparison.

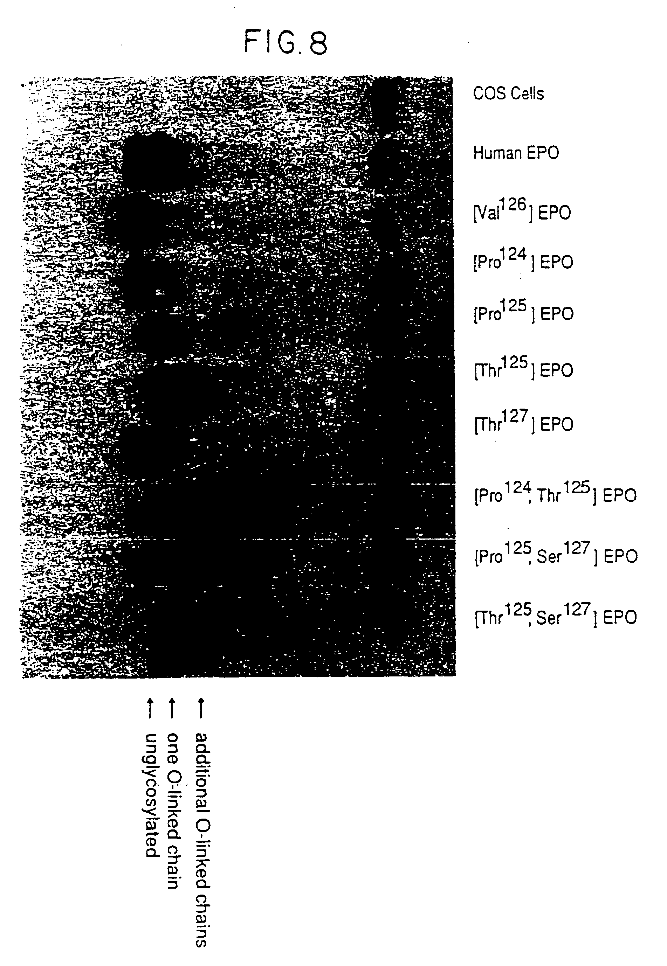

Figure 8 shows a Western blot analysis of COS cell supernatants of human sequence erythropoietin and indicated erythropoietin analogs after treatment with N-glycanase. The analogs [Thr¹²⁵] EPO and [Pro¹²⁴, Thr¹²⁵] EPO were constructed as described in Example 6. The analogs [Val¹²⁶] EPO, [Pro¹²⁴] EPO, [Pro¹²⁵] EPO, [Thr¹²⁷] EPO, [Pro¹²⁵, Ser¹²⁷] EPO and [Thr¹²⁵, Ser¹²⁷] EPO are shown for comparison.

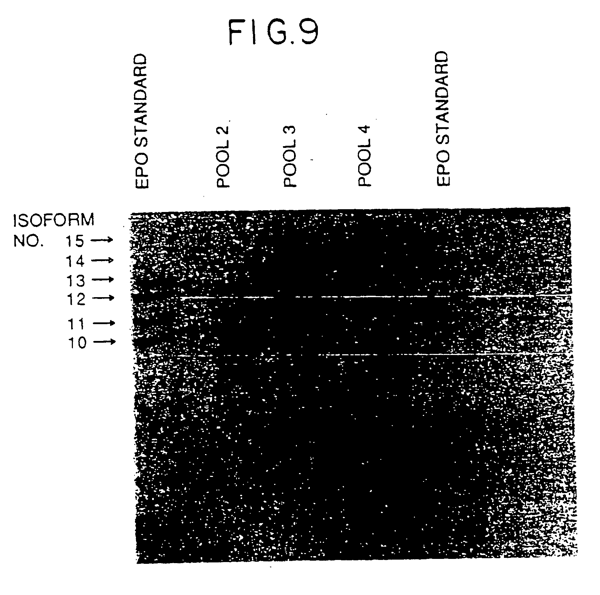

Figure 9 shows an isoelectric focusing gel of pools 2, 3 and 4 obtained by Q-Sepharose and C4 reverse phase chromatography of cell medium that supported the growth of CHO cells transfected with erythropoietin cDNA containing the [Thr¹²⁵] mutation. Purified recombinant erythropoietin containing a mixture of isoforms were obtained using procedures described in Example 2 of Lai et al., supra, except that DEAE-Agarose chromatography is replaced by Q-Sepharose chromatography, is also shown in the left and right lanes of the gel.

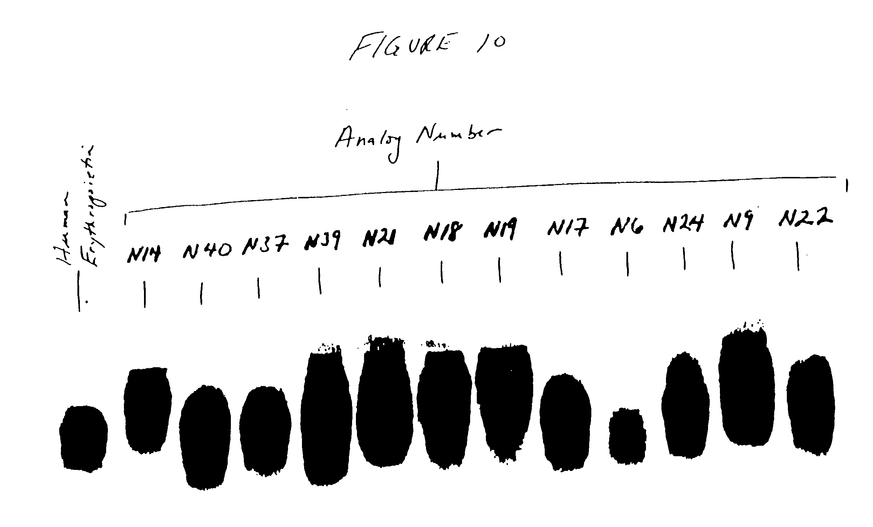

Figure 10 shows a Western blot analysis of COS cell supernatants of recombinant human erythropoietin (rHuEPO) and selected analogs. The construction of the analogs is described in Example 6. Analogs N9, N14, N18, N19, N21, N24 and N39 have at least one additional carbohydrate chain as evidenced by slower gel mobility.

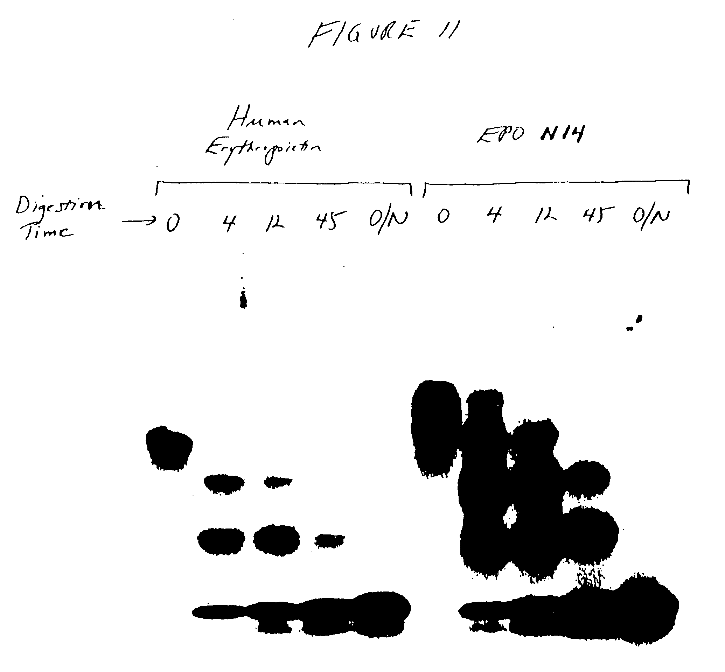

Figure 11 shows a Western blot analysis of COS cell supernatants of recombinant human erythropoietin and EPO N14 analog during N-glycanase digestion. Time points were taken at 0, 4, 12 and 45 minutes and after overnight digestion.

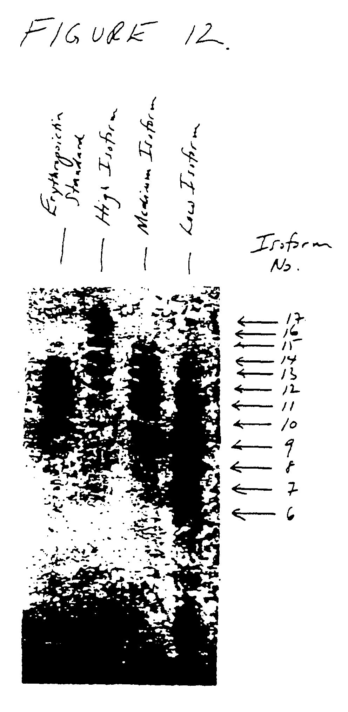

Figure 12 shows an isoelectric focusing gel of EPO N14 analog isoform preparations. The low isoform pool contains EPO N14 analog having mostly 6-12 sialic acids per molecule, the medium isoform pool contains analog N14 having mostly 10-15 sialic acids per molecule, and the high isoform pool contains EPO N14 analog having mostly 12-17 sialic acids per molecule.

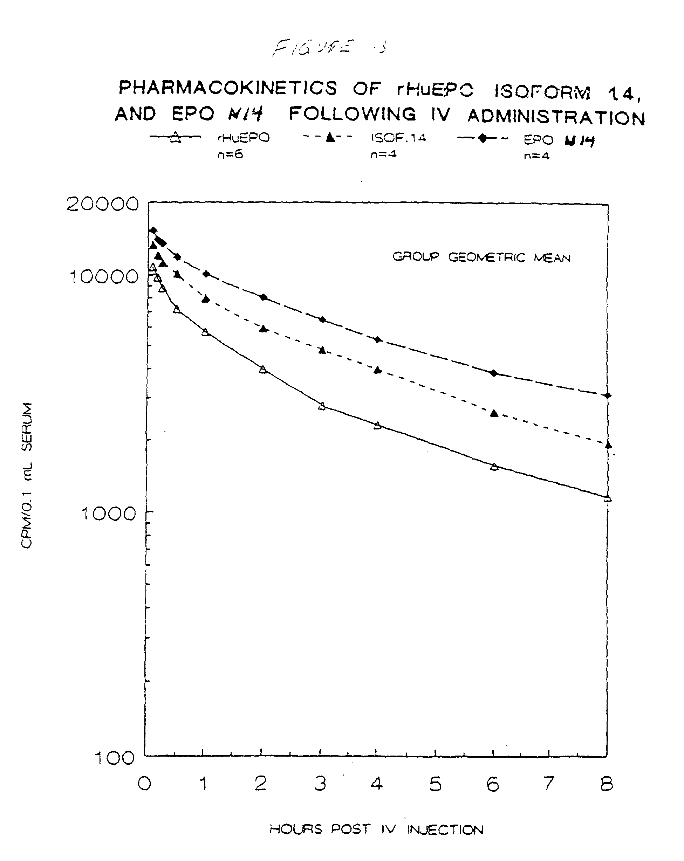

Figure 13 shows the pharmacokinetics of recombinant human erythropoietin, isolated isoform 14 and EPO N14 analog (isoforms 15-17) after intravenous injection into rats.

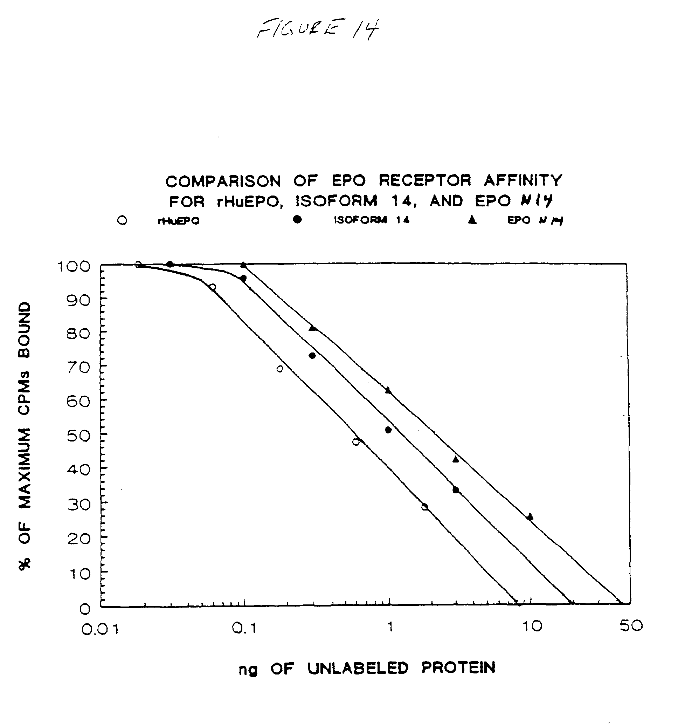

Figure 14 shows a cold displacement assay of ¹²⁵I labeled recombinant human erythropoietin binding to the erythropoietin receptor in the presence of varying amounts of unlabeled rHuEPO, isolated isoform 14 or EPO N14 analog.

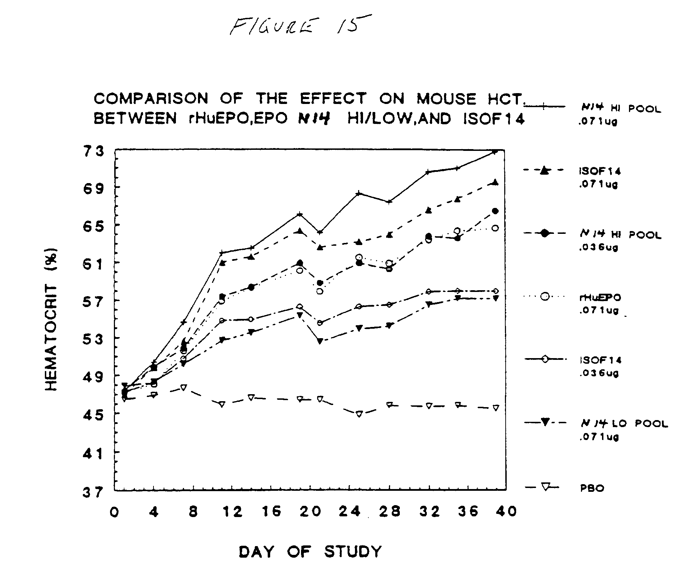

Figure 15 shows a mouse hematocrit study comparing the activity of EPO N14 high isoform pool (0.036 and 0.0712 µg), isolated isoform 14 (0.036 and 0.0712 µg), EPO 177 low isoform pool (0.0712 µg) and rHuEPO (0.071 µg).

### Detailed Description of the Invention

The subject invention provides erythropoietin isoforms. The specific isoforms of erythropoietin obtained in accordance with the present invention, and their properties, may vary depending upon the source of the starting material. For example, the isoforms of urinary derived human erythropoietin are different than the isoforms of recombinant erythropoietin: In a preferred embodiment, the invention relates to an erythropoietin isoform having a specific number (i.e. a fixed number greater than 0) of sialic acids per erythropoietin molecule, said number selected from the group consisting of 1-14. Advantageously said number is 9, 10, 11, 12, 13, or 14. In another embodiment, said number is greater than 14, advantageously 16-23.

The term "erythropoietin isoform" as used herein refers to erythropoietin preparations having a single isoelectric point (pl), and having the same amino acid sequence. The term "erythropoietin", as used herein, includes naturally occurring erythropoietin, urinary derived human erythropoietin as well as non-naturally occurring polypeptides having an amino acid sequence and glycosylation sufficiently duplicative of that of naturally occurring erythropoietin to allow possession of in vivo biological properties of causing bone marrow cells to increase production of reticulocytes and red blood cells.

It has been found that discrete isoforms of recombinant erythropoietin having the amino acid sequence of urinary derived human erythropoietin correspond to erythropoietin molecules having from 1-14 sialic acids, and each isoform present in purified recombinant erythropoietin has an in vivo activity which is related to the number of sialic acids the isoform possesses.

In a preferred embodiment, erythropoietin is the product of the expression of an exogenous DNA sequence that has been transfected into a non-human eucaryotic host cell, that is, in a preferred embodiment the erythropoietin is "recombinant erythropoietin". Recombinant erythropoietin is advantageously produced according to the procedures described in commonly owned Lin U.S. Patent 4,703,008 hereby incorporated by reference. Recombinant erythropoietin can be purified according to the general procedures described in Example 2 of commonly owned Lai et al. U. S. Patent 4,667,016 hereby incorporated by reference, or alternatively according to the procedure described in Example 2 of Lai et al. wherein DEAE-Agarose chromatography is replaced by Q-Sepharose chromatography.

Erythropoietin purified according to Example 2 of Lai et al. supra contains predominantly six isoforms when analyzed by IEF. In addition, at least one additional isoform of greater acidity has been detected using the chromatographic procedures described in Example 4. (This more acidic form, migrating at >14 sialic acids on an IEF gel may contain nonsialic acid negative charges as shown by the resistance of some of the charge to sialidase digestion). These isoforms differ from each other by sialic acid content. As shown in the Examples, this is demonstrated by isolating 10 of these isoforms by preparative IEF and determining the sialic acid content of five of them. Of the isoforms assayed for sialic acid content, it is found that the five isoforms contained either 9, 10, 11, 12 or 13 sialic acid residues.

There is a relationship between the relative in vivo specific activity of erythropoietin and number of sialic acid residues per erythropoietin molecule from the isoforms 5 through 11 (each isoform is designated herein by the number of sialic acids per erythropoietin molecule). Isoforms 11 through 14 have approximately the same relative in vivo specific activity. Isoforms 5-14 were assayed for in vivo activity by the exhypoxic polycythemic mouse bioassay and the amount of each isoform present is determined by Bradford protein assay, absorbance at 280 nm or by radioimmunoassay (RIA) for erythropoietin. RIA determinations (Egrie et al. Immunobiology 172, 213, (1986)), expressed as units/ml, are divided by 212,770 units/mg erythropoietin polypeptide, the average specific activity of purified erythropoietin as determined by RIA, to give protein concentrations of isolated isoforms or isoform mixtures expressed as mg erythropoietin polypeptide/ml. As shown in the Examples, the relative in vivo specific activities increase step-wise from isoform 5 to isoform 11 (see Table 2).

The in vivo specific activities referred to herein are measurements of relative in vivo specific activities and are not measurements of absolute in vivo specific activities. For the purposes of this application, the specific activities are used only to compare relative activities of isoforms that have been assayed using the same assay, using the same conditions including the same internal standard, the same type of animals, having the same analysis of the data used to calculate specific activity, the same assay for determining protein content. It is not intended that any in vivo specific activity value reported for any isoform represents an inherent or absolute value for that isoform.

This invention also provides compositions comprising two or more erythropoietin isoforms. In one embodiment the compositions comprise a mixture of isoforms having greater than a predetermined number of sialic acids per erythropoietin molecule, e.g. greater than 11 sialic acids per erythropoietin molecule, or greater than 12 sialic acids per molecule, e.g. a mixture of isoforms 12, 13 and 14. In another embodiment the compositions comprise mixtures of isoforms having a predetermined number of sialic acids per erythropoietin molecule, e.g. less than 12, but greater than 8 sialic acids per molecule as in, for example, a mixture of isoforms 9, 10, and 11. The invention also provides for compositions of erythropoietin isoforms wherein the relative amounts of the isoforms are the same or different. For example, a mixture of isoforms 9, 10 and 11 could have the isoforms present in a variety of ratios such as 1:1:1, 2:3:1 or 20:20:1.

Advantageously, the compositions comprise mixtures of less than four isoforms, for example a mixture of isoforms 11, 12, and 13, or a mixture of 12 and 14, or a mixture of 7 and 13.

In order to produce mixtures of erythropoietin isoforms, this invention also provides methods of isolating selected erythropoietin isoforms simultaneously. These methods include isolation of individual isoforms by techniques such as preparative isoelectric focusing or preparation of mixtures of isoforms having a predetermined number of sialic acids per molecule (for example, greater than 11) by techniques such as ion exchange chromatography or chromatofocusing. All of these techniques have as their basis the separation of proteins according to charge.

In general, ion exchange chromatography and chromatofocusing involve application of either crude human erythropoietin (cell conditioned media) or purified material to a column resin under conditions that permit binding of some or all of the erythropoietin isoforms to the resin. For crude erythropoietin preparations, it is preferable to apply the protein to the column at about pH 7 while for purified preparations the protein can be applied to the column at pH 7 down to about pH 4. After washing the column with buffer at about pH 4, those erythropoietin isoforms that remain bound on the ion exchange column are eluted by increasing the pH and the salt concentration of the buffer or by applying a gradient of decreasing pH and increasing ionic strength at about pH 4. For chromatofocusing, the isoforms are eluted from the column by a gradient of decreasing pH or by washing the column with a high concentration of salt.

In a preferred embodiment, individual isoforms are isolated using ion exchange chromatography. As an example, isoform 14 was isolated using ion exchange chromatography as described in Example 8.

Also encompassed by the invention are certain analogs of human erythropoietin. As used herein the phrase "analog of human erythropoietin" refers to erythropoietin with one or more changes in the amino acid sequence of human erythropoietin which result in an increase in the number of sites for sialic acid attachment. Analogs are generated by site-directed mutagenesis having additions, deletions, or substitutions of amino acid residues that increase or alter sites that are available for glycosylation. Such analogs may have a greater number of carbohydrate chains than human erythropoistin.

The erythropoietin analogs of the present invention comprise an amino acid sequence which includes at least one additional site for glycosylation. Analogs having levels of sialic acid greater than those found in human erythropoietin are generated by adding glycosylation sites which do not perturb the secondary or tertiary conformation required for biological activity. Advantageously, the analog of human erythropoietin has 1, 2 or 3 additional sites for N-glycosylation or O-glycosylation, resulting in the addition of 1, 2, or 3 additional N-linked or O-linked carbohydrate chains. For example, a leucine at position 69 is replaced by an asparagine to give the sequence Asn-Leu-Ser, which serves as a fourth site for N-glycosylation. Such a change can commonly provide up to four additional sialic acids per molecule. Examples of changes that generate additional O-glycosylation sites are alanine at position 125 to threonine and alanines at positions 124 and 125 to proline and threonine, respectively. Analogs may be constructed which have one or more additional N-linked and O-linked chains, for example, analogs NO1 and NO2 described in Table 5. As will be appreciated by those skilled in the art, the subject invention includes many other analogs of human erythropoietin having additional sites for glycosylation.

Analogs having increased levels of carbohydrate attachment at a glycosylation site are also encompassed by the invention. Such analogs usually involve the substitution of one or more amino acids which are in close proximity to an N-linked or O-linked site. As a result of these amino acid changes, a greater proportion of erythropoietin polypeptides will have a carbohydrate modification. The glycosylation sites may be naturally occurring or generated by mutation. For example, analog N13 does not generate an additional carbohydrate chain even though a glycosylation site was introduced at position 88. However, analogs N14 and N18, which have serine and valine residues, respectfully, substituted at position 87, have an additional carbohydrate chain at position 88.

Also provided by the invention are analogs which have one or more amino acids extending from the carboxy terminal end of erythropoietin and wherein the carboxy terminal extension provides at least one additional carbohydrate site. In one embodiment, an analog was constructed by fusing the carboxy-terminal 28 amino acids of human chorionic gonadotropin (HCG) to the arginine residue at position 166 of human erythropoietin. The HCG carboxy-terminal fragment has four sites for O-glycosylation (Kessler et al., J. Biol. Chem. 254, 7907 (1979)).

Tables 3, 4 and 5 list erythropoietin analogs which have additional sites for N-linked and/or O-linked carbohydrate chains. The analogs have the sequence Asn-X-Ser/Thr substituted at various positions in the human erythropoietin polypeptide chain to create N-linked sites or have serine or threonine residues introduced to create O-linked sites.

Table 6 lists those analogs which add at least one additional N-linked or one additional O-linked carbohydrate chain, or add additional N-linked and O-linked chains simultaneously, as evidenced by the migration of the glycoproteins on SDS gels (Example 7). As can be appreciated from Tables 3-6, analogs having one or more additional sites for carbohydrate attachment do not necessarily result in erythropoietin molecules having additional carbohydrate chains. For example, substitution of threonine residues at positions 123 and 125 resulted in the addition of an O-linked carbohydrate chain while substitution of serine or threonine at other positions did not result in analogs with additional O-linked chains (see Table 4). However, substitution of asparagine residues at positions 30, 51, 57, 69, 88, 89, 136 and 138 in the human erythropoietin amino acid sequence resulted in the addition of an N-linked chain at those sites. The fusion of an HCG polypeptide fragment to the arginine residue at position 166 of human erythropoietin resulted in an erythropoietin-HCG fusion molecule having at least two additional O-linked carbohydrate chains.

The erythropoietin analogs of the present invention also encompass erythropoietin having an amino acid sequence which includes a rearrangement of at least one site for glycosylation. A rearrangement of a glycosylation site as used herein refers to the deletion of one or more glycosylation sites in human erythropoietin and addition of one or more non-naturally occurring glycosylation sites. Analogs R1, R2 and R3 are examples of such rearrangements and were constructed by deletion of the N-linked sites at positions 24, 38 or 83, respectively, and addition of an N-linked site at position 88. However, numerous other types of carbohydrate site rearrangements are possible and the resulting analogs may or may not have a greater number of glycosylation sites compared to human erythropoietin.

Analogs R1, R2 and R3 were analyzed for in vivo biological activity and the results shown in Table 7. The introduction of an N-linked chain at Asn 88 restored biological activity to erythropoietin having any one of the three naturally occurring N-linked sites deleted. These results indicate that the positions of carbohydrate chains in erythropoietin may be changed in order to generate useful analogs without significantly affecting the biological activity.

Also encompassed by the present invention are DNA sequences encoding erythropoietin analogs having additional sites for N-linked and/or O-linked chains, analogs having a rearrangement of at least one attachment site for a carbohydrate chain, and analogs having one or more amino acids extending from the carboxy-terminal end of erythropoietin. Procedures used to introduce changes into the human erythropoietin DNA sequence for the purpose of creating and altering attachment sites for carbohydrates are disclosed in Example 6.

These erythropoietin analogs can be the product of expression of an exogenous DNA sequence, i.e., produced through recombinant DNA technology, or can be synthesized products. An exogenous DNA sequence comprises cDNA, genomic DNA or chemically synthesized DNA encoding an erythropoietin analog. Recombinant DNA plasmids and eucaryotic host cells useful for the expression of said analogs are also provided. Expression vectors include any vector which is capable of expressing cloned DNA sequences in a eucaryotic host cell, particularly those vectors used for expression in COS and CHO cells. Examples of such vectors include plasmids pEC and pDECΔ described in Example 6 of the specification. The cultivation of COS and CHO host cells expressing erythropoietin analogs was carried out using procedures known to one skilled in the art.

Isolated isoforms and mixtures of isoforms derived from erythropoietin analogs are obtained using the methods described above for preparing isoforms of human erythropoietin. These methods may include isoelectric focusing, ion exchange chromatography and chromatofocusing. Preferably, ion exchange chromatography is used to prepare individual isoforms and mixtures of isoforms derived from erythropoietin analogs.

Increasing the number of carbohydrate chains on erythropoietin, and therefore the number of sialic acids per erythropoietin molecule, may confer advantageous properties such as increased solubility, greater resistance to proteolysis, reduced immunogenicity, increased serum half-life, and increased biological activity.

Conditioned media from CHO cells expressing erythropoietin analogs were analyzed for in vivo biological activity and the results shown in Table 6. Several analogs tested had activity that was 3-fold or more higher than human erythropoietin. In particular, analogs having an additional N-linked carbohydrate chain at either position 30 or 88 show 2- to 3-fold higher activity than human erythropoietin while analogs having additional O-linked chains as a result of fusion of human erythropoietin to the HCG polypeptide fragment have at least 2-fold higher activity.

Two erythropoietin analogs having additional carbohydrate chains were purified and isoform mixtures having different sialic acid contents were isolated (Example 8). Thr¹²⁵ and Ser⁸⁷Asn⁸⁸Thr⁹⁰ (EPO N14) analogs were separated into three separate isoform fractions and the in vivo biological activities for each fractions was determined. The results presented in Table 8 demonstrate that EPO N14 isoform fractions having higher sialic acid content have a greater in vivo activity.

A pool of high sialic acid isoforms of EPO N14 analog and recombinant human erythropoietin (isoforms 9-14) were studied in receptor binding assays, pharmacokinetic experiments, and experiments to determine increase in hematocrit of mice. Results of these assays indicate that there is a direct relationship between sialic acid content, clearance half-life, and ability to increase the hematocrit of treated mice. Thus, as shown in figures 13, 14 and 15, EPO N14 high sialic acid isoform pool had a significantly longer in vivo halt-life and promoted a greater increase in hematocrit than did either isolated isoform 14 or recombinant human erythropoietin, even though the N14 high sialic acid isoform pool did not bind as strongly to the receptor.

Another embodiment of the invention relates to mammalian (e.g., Chinese Hamster Ovary, CHO) host cells which preferentially synthesize isoforms of human erythropoietin or erythropoietin analogs having greater than a specific number of sialic acids per molecule, e.g. greater than 10 sialic acids per molecule. Erythropoietin molecules have N-linked and O-linked oligosaccharides structures which can limit the sialic acid content of the molecule. For example, tetraantennary (four-branched) N-linked oligosaccharides most commonly provide four possible sites for sialic acid attachment while bi- and triantennary oligosaccharide chains, which can substitute for the tetraantennary form at asparagine-linked sites, commonly have at most only two or three sialic acids attached. O-linked oligosaccharides commonly provide two sites for sialic acid attachment. Thus, erythropoietin molecules can accommodate a total of 14 sialic acid residues provided all three N-linked oligosaccharides are tetraantennary. Mammalian cell cultures are screened for those cells that preferentially add tetraantennary chains to recombinant erythropoietin, thereby maximizing the number of sites for sialic acid attachment.

The N-linked oligosaccharides of urinary erythropoietin contain sialic acid in both an α 2,3 and an α 2,6 linkage to galactose (Takeuchi et al. J. Biol. Chem. 263, 3657(1988)). Typically the sialic acid in the α 2,3 linkage is added to galactose on the mannose α 1,6 branch and the sialic acid in the α 2,6 linkage is added to the galactose on the mannose α 1,3 branch. The enzymes that add these sialic acids (β-galactoside α 2,3 sialyltransferase and β-galactoside α 2,6 sialyltransferase) are most efficient at adding sialic acid to the mannose α 1,6 and mannose α 1,3 branches respectively.

Dihydrofolate reductase (DHFR) deficient Chinese Hamster Ovary (CHO) cells are a commonly used host cell for the production of recombinant glycoproteins including recombinant erythropoietin. These cells do not express the enzyme β-galactoside α 2,6 sialyltransferase and therefore do not add sialic acid in the α 2,6 linkage to N-linked oligosaccharides of glycoproteins produced in these cells. (Mutsaers et al. Eur. J. Biochem. 156, 651 (1986); Takeuchi et al. J. Chromatogr. 400, 207 (1987)). Consequently, recombinant erythropoietin produced in CHO cells lacks sialic acid in the 2,6 linkage to galactose (Sasaki et al. (1987), supra; Takeuchi et al. (1987), supra).

In another embodiment of the subject invention, human erythropoietin or an erythropoietin analog is produced in CHO cells that are transfected with a functional β-galactoside α 2,6 sialyltransferase gene to give incorporation of sialic acid in α 2,6 linkage to galactose. The resulting isoforms will contain sialic acid having both α 2,3 and α 2,6 linkages to galactose. See Lee et al. J. Biol. Chem. 264, 13848 (1989), hereby incorporated by reference, for a disclosure of techniques for creating modified CHO cells or other mammalian host cells.

Also comprehended by the invention are pharmaceutical compositions comprising a therapeutically effective amount of a specific isoform or mixture of isoforms together with a suitable diluent, adjuvant and/or carrier useful in erythropoietin therapy. Pharmaceutical compositions comprising a therapeutically effective amount of an erythropoietin analog together with a suitable diluent, adjuvant and/or carrier are also encompassed. A "therapeutically effective amount" as used herein refers to that amount which provides therapeutic effect for a given condition and administration regimen. The administration of isoforms of human erythropoietin or erythropoietin analogs is preferably by parenteral routes. The specific route chosen will depend upon the condition being treated. The administration of isoforms of human erythropoietin or erythropoietin analogs is preferably done as part of a formulation containing a suitable carrier, such as human serum albumin, a suitable diluent, such as a buffered saline solution, and/or a suitable adjuvant. The required dosage will be in amounts sufficient to raise the hematocrit of patients and will vary depending upon the severity of the condition being treated, the method of administration used and the like.

The following examples are offered to more fully illustrate the invention, but are not to be construed as limiting the scope thereof. The erythropoietin standard used in the in vivo bioassays employed in the Examples is a recombinant erythropoietin standard that was standardized against a partially purified urinary erythropoietin standard. Thus, only relative in vivo specific activities are being measured. Also the in vivo specific activities are expressed in "units/ml", "units/mg" and units/A₂₈₀" and not as "IU/ml", "IU/mg" and IU/A₂₈₀" because the erythropoietin standard employed has not been directly correlated to any existing international standard.

### Example 1: Isolation of Recombinant Erythropoietin Isoforms

Recombinant erythropoietin is produced as described in Lin, supra. Recombinant erythropoietin used as starting material for the first and third isoform isolations is purified according to the procedure described in Example 2 of commonly owned Lai et al., supra. Starting material for the second and fifth isoform isolation is purified according to Lai et al. supra using the modification of Q-Sepharose chromatography. These preparations contain a mixture of isoforms of recombinant erythropoietin having the same amino acid sequence as urinary derived human erythropoietin and contain predominantly isoforms 9 to 14. Starting material for the fourth isoform preparation is the material which elutes during the 5 mM acetic acid/1 mM glycine/6M urea wash of the anion exchange column in Example 2 of Lai et al. This fraction contains isoforms with less than or equal to 9 sialic acids and was further purified by gel filtration chromatography as described in Example 2 of Lai et al. prior to use in the preparative isoelectric focusing procedure. The sixth isoform preparation used as its starting material a purified preparation of recombinant erythropoietin having from 4 to 13 sialic residues. This material was purified as per Example 2 of Lai et al. except for a modification to the ion exchange column (elution of the recombinant erythropoietin with a sodium chloride gradient at pH 8.4 and omission of the acetic acid/urea wash) which results in retention of most of the isoforms present in the starting material.

Six different preparations of individual isoforms are carried out by preparative isoelectric focusing in a granulated gel bed (Ultrodex, LKB) essentially as per LKB Application Note 198. Pharmalyte (Pharmacia) 2.5-5 ampholytes (Pharmacia) are used and the gel bed contains 5 M urea.

In the first preparation, approximately 20 mg of recombinant erythropoietin in 6.8 ml of 20 mM sodium citrate/ 100 mM sodium chloride, pH 7.0 are applied to the gel and focused at 8 watts for approximately 16 hours. After isoelectric focusing, the isoform bands in the gel are visualized by a paper contact print of the gel bed. The print is made and then fixed by soaking in 3 changes (approximately 10 minutes each, room temperature) of fixing solution (40% methanol/10% acetic acid/10% TCA/3.5% sulfosalicylic acid), subjected to one change (approximately 10 minutes) of 40% methanol/10% acetic acid (30-60°C), stained for 15 minutes at 60°C in 0.125% Coomassie Blue R-250/40% methanol/10% acetic acid, and then destained in 7.5% methanol/10% acetic acid in order to visualize the separated isoforms. The region of the granulated gel bed containing the isoforms (∼50% of the resin) is removed, water is added (∼16 ml), and the slurry is poured into a 5.5 x 24.5 inch tray and evaporated to ∼40 g net weight. This preparation is focused for a second time and a contact print of the gel bed is made as before. The portion of gel containing each of the six discernible isoforms is removed from the gel bed.

In order to elute the isoforms from the gel, a solution containing 10 mM Tris-HCI, pH 7.0/ 5 mM Chaps is added to each isoform to generate a slurry. The slurries are placed in small columns and washed with the Tris-Chaps buffer. The flow throughs are collected and applied separately to small columns (open column configuration) containing Vydac C4 reversed phase resin equilibrated in 20% ethanol/10 mM Tris-HCl, pH 7.0. The columns are developed stepwise with 20% ethanol/10 mM Tris-HCl, pH 7.0, 35% ethanol/10 mM Tris-HCl, pH 7.0, and 65% ethanol/10 mM Tris-HCl, pH 7.0. The fraction eluting at 65% ethanol/10 mM Tris is diluted 1:1 with 10 mM Tris-HCl, pH 7.0 and subjected to concentration and then buffer exchanged to 10 mM Tris-HCl, pH 7.0 using a Centricon-10 (Amicon) microconcentrator. Analytical isoelectric focusing of this preparation is performed essentially as described in LKB technical note 250 using Servalyte 3-5 ampholines (Serva) in a polyacrylamide gel containing 5 M urea.

In a second preparation, approximately 26 mg of recombinant erythropoietin in 6.5 ml of deionized water are applied to the gel and focused at 2.5 watts for 35 minutes and 10 watts for approximately 17 hours. The bands of focused protein, which are visible in the gel bed, are removed as 11 different pools. Each pool is brought to about 7.5 ml with deionized water and 20 ml of each of the resulting pool supernatants is subjected to analytical isoelectric focusing as described above. To each of the pools is added 5 ml of 1.5 M Tris-HCl, pH 8.8 and the slurries are each placed in small columns and the liquid phase allowed to flow through. The resin is washed with approximately three volumes of 0.5 M Tris-HCl, pH 7 and the rinse solution is combined with the flow through. The eluants are concentrated and buffer exchanged to 20 mM sodium citrate/ 100 mM sodium chloride, pH 7.0 using Amicon disposable ultrafiltration devices having a 10,000 dalton molecular weight cutoff. The concentrated solutions (approximately 0.5 ml) are then passed through a 0.22 micron cutoff cellulose acetate filter. Based upon analytical isoelectric focusing, five pools are found to contain predominantly the single isoforms 10, 11, 12, 13 and 14.

In a third preparation, approximately 30 mg of recombinant erythropoietin in 21.8 ml of distilled water is applied to the gel and focused at 2 watts for 25 minutes, 10 watts for 20 hours and 15 watts for 15 minutes. Protein bands corresponding to the individual isoforms are observed visually and removed from the gel bed. Distilled water is added to gel-isolated isoforms to generate a slurry and the resulting supernatants are analyzed by analytical isoelectric focusing. An equal volume of 1 M Tris-HCl, pH 7.2 is added to each slurry, the suspensions are placed into separate small columns, and the liquid phase is allowed to flow through the column to elute the isoforms. Each flow through is concentrated and buffer exchanged to 20 mM sodium citrate/ 100 mM sodium chloride, pH 7.0 using Amicon disposable ultrafiltration devices having a 10,000 dalton molecular weight cutoff. An analytical isoelectric focusing gel revealed that pools containing predominantly the single isoforms 9, 10, 11, 12, 13 and 14 were obtained.

A fourth isoform preparation used as its starting material erythropoietin containing isoforms 3-9 (prepared as described above). Prior to preparative isoelectric focusing carried out essentially as described for preparations 1-3 above, the ampholytes (Pharmalyte 2.5-5) were pre-fractionated in a Rotofor (Bio-Rad, Richmond, CA) liquid phase isoelectric focusing cell to yield an ampholyte range more suitable for the lower isoelectric points of the starting material. The prefractionation was carried out by mixing 6.7 mL of Pharmalyte 2.5-5 with 15 g of urea and adding purified water to bring the volume to 50 mL. This mixture was fractionated in the Rotofor at 10 Watts, 1°C, for 5 1/2 hours using 0.1 M phosphoric acid and 0.1 M sodium hydroxide as the anolyte and catholyte, respectively. The ampholyte fractions having measured pHs of between 4.5 and approximately 6 were used in the flat-bed isoelectric focusing.

Ampholytes were removed from the isoforms using a Centrieluter (Amicon, Danvers, MA) and a 10,000 MW cutoff Centricon (Amicon) using the following parameters: 0.18 Tris buffer pH 8.8, 100 Volts, 25-30 mA, for 3 hours. The isoforms were then buffer exchanged into 0.1 M sodium chloride by gel filtration using Sephadex G-25 (Pharmacia). Analytical isoelectric focusing of the five resulting pools showed them to contain isoforms 4,5,6,7, and 8. Isoform 4 ran as several bands, indicating that it may have undergone some degradation.

The fifth isoform preparation was modified by the addition of a pre-focusing step to the flat bed isoelectric focusing procedure. In this modification, the protein was not added to the ampholyte/urea/gel mixture prior to electrophoresis but was added to the isoelectric focusing apparatus following generation of the pH gradient in the gel bed. Following prefocusing for 75 minutes (1500 volt-hrs) the section of gel bed from 2.25-4.25 cm from the cathode was removed, mixed with the erythropoietin solution, and added back to the gel bed. Following isoelectric focusing, isoforms 10,11,12,13 and 14 were eluted from the gel bed and separated from the ampholytes by ultrafiltration using Centricon-10 (Amicon) devices.

The pre-focusing modification was undertaken to make the ultraviolet absorbance characteristics of the isoform preparations more similar to that of the starting recombinant erythropoietin. This improvement in spectral characteristics can be seen in the ratio of absorbance at 280 and 260 nm for the isolated isoforms. The average ratio of absorbance at 280 nm to that at 260 nm (A₂₈₀/A₂₆₀) for isoforms from preparations 2 and 3 (non-prefocused) is 1.36 ± 0.11 while the average A₂₈₀/A₂₆₀ ratio for preparations 5 and 6 (pre-focused) is 1.68 ± 0.20. When isoform #14 is excluded from the calculation, the average A₂₈₀/A₂₆₀ ratios are 1.39 ± 0.11 and 1.74 ± 0.09 for preparations 2 & 3 and 5 & 6, respectively. (Isoform 14 may have the most atypical spectrum because it is present in the smallest amounts and is thus more subject to interferences by trace contamination by ampholyte components or because it is nearest to the electrode during the flat bed isoelectric focusing procedure). The average A₂₈₀/A₂₆₀ ratio for recombinant erythropoietin prepared according to Example 2 of Lai et al. (modified as described earlier by using Q-Sepharose as the anion exchange resin) is 1.91 ± 0.04.

As described above, the starting material for isoform preparation #6 was a recombinant erythropoietin preparation containing isoforms 4-13. The ampholytes were pre-focused in the Rotofor apparatus as per the fourth preparation. Ampholyte fractions having measured pHs of between 3.7 and 4.8 were used for the flat bed isoelectric focusing. The flat bed was pre-focused as in run #5 and isoforms 9,10,11,12 and 13 were obtained after ultrafiltration (Centricon-10) to remove carrier ampholytes.

### Example 2: Sialic Acid Content of Recombinant Erythropoietin Isoforms

The isoforms isolated as described in Example 1 and erythropoietin purified according to procedures described in Lai et al., supra (mixture of isoforms 9 to 14) are buffer exchanged into 0.10-0.15 M sodium chloride and analyzed for sialic acid content by a modification of the procedure of Jourdian et al. J. Biol. Chem. 246, 430 (1971). The sialic acid residues are cleaved from the glycoproteins by hydrolysis with 0.35 M sulfuric acid at 80°C for 30 minutes and the solutions are neutralized with sodium hydroxide prior to analysis. In order to estimate the amount of erythropoietin protein present, a Bradford protein assay (Bradford Anal. Biochem. 72, 248 (1976)) using recombinant erythropoietin having the amino acid sequence of human erythropoietin as standard is performed using the assay reagents and the micro-method procedure supplied by Bio-Rad. The results, expressed as moles of sialic acids per mole of erythropoietin, are shown in Table 1. Isoforms are designated according to the number of sialic acids per molecule and range from least acidic (Isoform 9) to most acidic (Isoform 13). Isoforms 9-13 are shown in gel lanes 6-10 of Figure 1. Quantities of Isoform 14 are insufficient to accurately measure the sialic acid content. The sialic acid content of this isoform is inferred from its migration on IEF gels relative to other isoforms. The sialic acid content of isoforms 5-8 (preparation #4) has not been measured but is likewise inferred from their migration on IEF gels.

**TABLE 1**

| ERYTHROPOIETIN ISOFORM | MOLES SIALIC ACID/MOLE ERYTHROPOIETIN |
|---|---|
| Isoform 13 | 12.9 ± 0.5 |
| Isoform 12 | 11.8 ± 0.2 |
| Isoform 11 | 11.0 ± 0.2 |
| Isoform 10 | 9.8 ± 0.3 |
| Isoform 9 | 8.9 ± 0.6 |
| Isoform Mixture (9-14) | 11.3 ± 0.2 |

### Example 3: Activity of Recombinant Erythropoietin Isoforms

The isoforms isolated as described in Example 1 are assayed by absorbance at 280 nm, by Bradford protein assay and by RIA for erythropoietin to determine the amount of recombinant erythropoietin present. The exhypoxic polycythemic mouse bioassay (Cotes et al. Nature 191, 1065 (1961)) is used to determine the relative in vivo biological activity. Quantitation of the amount of erythropoietin protein present using a radioimmunoassay for erythropoietin produced results having higher relative in vivo specific activity for certain isoforms because of an apparent decreased immunoreactivity of isoforms containing large amounts of sialic acid leading to an underestimation of the erythropoietin concentration and thus an overestimation of the relative in vivo specific activity for the most negative isoforms. Mouse bioassay determinations, expressed as units/ml, are divided by the corresponding protein concentrations to give in vivo specific activities expressed as units/mg erythropoietin polypeptide. These specific activities are shown in Table 2.

In Table 2, "n" is the number of independent isoform preparations which contribute to the specific activity value. In most cases several in vivo assays were performed on each isoform preparation. The same in vivo data contribute to the specific activity calculations for all three columns, units/mg erythropoietin polypeptide was determined by the absorbance at 280 nm, from radioimmunoassay potencies, or from Bradford protein assay results. Purified recombinant erythropoietin containing isoforms 9-14 was used as the standard in the Bradford protein assay. "n" may be less for the calculation made using the Bradford protein assay as some preparations were no longer available at the time the Bradford assays were performed.

Erythropoietin purified according to the procedures described in Lai et al., supra and containing a mixture of isoforms 9 to 14 is used as a standard for the RIAs and in vivo assays.

The relative specific activities expressed as units/mg erythropoietin polypeptide can be converted to units/A₂₈₀ by multiplying by 0.807 mg erythropoietin polypeptide/A₂₈₀. The conversion factor is derived by multiplying the extinction coefficient of erythropoietin (1.345 mg/A₂₈₀) by the protein content of the erythropoietin glycoprotein (about 60% by weight, Davis et al. Biochemistry 26, 2633 (1987)) to obtain mg erythropoietin polypeptide/A₂₈₀ (i.e., 1.345 mg erythropoietin/A₂₈₀ x 0.60 mg polypeptide/mg erythropoietin = 0.807 mg polypeptide/A₂₈₀). In addition, specific activities expressed as units/mg erythropoietin polypeptide can be multiplied by the factor 0.60 mg polypeptide/mg erythropoietin glycoprotein to give specific activities expressed as units/mg erythropoietin glycoprotein.

**TABLE 2**

| Isoform | U/mG Polypeptide (Bradford Protein Assay) | n | U/mG Polypeptide (From A280) | n | U/mG Polypeptide (From RIA) | n |
|---|---|---|---|---|---|---|
| 14 | 289,400 ± 3,100 | 2 | 205,800 ± 37,700 | 2 | 366,700 ± 55,900 | 2 |
| 13 | 307,600 ± 30,600 | 4 | 258,700 ± 59,500 | 5 | 337,200 ± 40,200 | 5 |
| 12 | 275,200 ± 55,600 | 4 | 258,400 ± 41,700 | 5 | 287,700 ± 42,600 | 5 |
| 11 | 282,700 ± 41,100 | 3 | 255,800 ± 67,300 | 4 | 251,400 ± 62,700 | 4 |
| 10 | 188,000 ± 1,900 | 1 | 170,300 ± 34,500 | 3 | 171,900 ± 31,600 | 3 |
| 9 | - | | 96,600 ± 46,700 | 2 | 113,600 ± 39,600 | 2 |
| 8 | 65,200 ± 3,800 | 1 | 70,600 ± 4,100 | 1 | 61,000 ± 3,500 | 1 |
| 7 | 46,200 ± 5,800 | 1 | 50,300 ± 6,300 | 1 | 42,800 ± 5,400 | 1 |
| 5 | 16,600 ± 1,700 | 1 | 18,300 ± 1,900 | 1 | 15,500 ± 1,600 | 1 |

The data in Table 2 are also presented graphically in Figures 2A, 2B and 2C. These data show that the relative in vivo activity of erythropoietin increases as a function of sialic acid content up until isoform #11. Isoforms 11-14 have essentially the same relative in vivo bioactivity. (This is most apparent when the concentration of isoform 14 is expressed using the Bradford assay value. The Bradford value may be more accurate for isoform 14 because of the generally low levels obtained and the resulting difficulty in determination by A₂₈₀ and the most apparent decreased reactivity in the RIA of very negative forms discussed previously). The greater relative in vivo specific activity of erythropoietin isoforms having more sialic acid is most likely due to a longer circulating half-life of these forms. Isoforms 9 and 13 were labeled with radioactive iodine (¹²⁵I) and their rate of clearance in rats was determined. The half-life in circulation was significantly longer for isoform 13 than for isoform 9.

### Example 4: Selection of Recombinant Erythropoietin Isoform Mixtures by O-Sepharose Chromatography

Cell conditioned media from the production of recombinant erythropoietin according to the procedures described in Lin, supra are concentrated and diafiltered against 10 mM Tris, pH 7.2. Protein concentration is determined by the Bradford microprotein assay using bovine serum albumin as a standard. 19.6 ml of the solution containing 40 mg of total protein is made 20 µM in CuS0₄, filtered through a 0.45 micron cutoff filter and loaded onto a 4 ml bed volume (1.05 cm height x 2.2 cm diameter) column packed with Q Sepharose Fast Flow (Pharmacia) which has been equilibrated with 10 mM Tris, pH 6.8 to 7.0 at 4°C. After sample application, the column is washed with two column volumes of the same buffer. The column flow rate is about 1 ml/min. Six separate columns are set up using this procedure to select defined erythropoietin isoform mixtures.

Columns are washed with 6 to 9 column volumes of a low pH buffer consisting of: Column #1, 150 mM acetic acid, 1 mM glycine, 20 µM CuSO₄, 6 M urea adjusted to pH 4.7 with NaOH; Column #2, 200 mM acetic acid, 1 mM glycine, 20 µM CuSO₄, 6 M urea adjusted to pH 4.7 with NaOH; Column #3, 250 mM acetic acid, 1 mM glycine, 20 µM CuSO₄, 6 M urea adjusted to pH 4.7 with NaOH; Column #4, 300 mM acetic acid, 1 mM glycine, 20 µM CuSO₄, 6 M urea adjusted to pH 4.7 with NaOH; Column #5, 150 mM acetic acid, 1 mM glycine, 20 µM CuSO₄, 6 M urea; Column #6, 300 mM acetic acid, 1 mM glycine, 20 µM CuSO₄, 6 M urea. The pH of the columns is increased to approximately pH 7 by washing each one with 8 to 11 column volumes of 10 mM Tris-HCl, 55 mM NaCl, 20 µM CuSO₄, pH 7. The defined erythropoietin isoform mixtures are eluted from the columns by washing with 10 mM Tris-HCl, 140 mM NaCl, 20 µM CuSO₄, pH 7.0.

The eluted isoform pools from each column are concentrated and solvent exchanged into water using an Amicon Centricon-10 microconcentrator. The results of analytical isoelectric focusing of these concentrated pools are shown in Figure 3. Gel lanes 1-6 represent defined erythropoietin isoform mixtures eluted from column 1-6, respectively. The "isoform mixture" shown in the far right gel lane of Figure 3 represents cell media which is applied to a Q-Sepharose column as described above, the column is washed with 5 mM acetic acid, 1 mM glycine, 20 µM CuSO₄, 6M urea, and the erythropoietin isoform mixture is eluted from the column using the procedures described above. This eluted mixture of isoforms is further purified according to the procedures described in Lai et al., supra prior to analytical isoelectric focusing.

### Example 5: Fractionation of Recombinant Erythropoietin Isoforms Using a Low pH Gradient on O-Sepharose

In another procedure, erythropoietin isoforms are separated using a gradient of decreasing pH and increasing ionic strength. The concentrated diafiltered erythropoietin containing media is loaded to a column of Q-Sepharose at a ratio of approximately 40 mg total protein/mL gel. The column is then washed with approximately two column volumes of 10 mM Tris HCl, pH 7.0 and then approximately 10 column volumes of 2 mM acetic acid/1 mM glycine/20 µM CuSO₄/6 M urea (pH approximately 4.8) to remove contaminating proteins and erythropoietin isoforms containing less than approximately 7 sialic acid residues. Isoforms containing from approximately 8 to approximately 12 sialic acids are eluted from the column using a gradient starting at approximately 2 mM acetic acid in 6 M urea/1 mM glycine/20 µM CuSO₄ and running to 40 mM acetic acid/6 M urea/1 mM glycine/20 µM CuSO₄ (pH approximately 4). The total volume of the gradient is approximately 40 column volumes and fractions of approximately one column volume each are collected into vessels containing a volume of Tris buffer sufficient to bring the pH into the range of 6-8.5 so as to avoid long term exposure of the collected fractions to low pH. Aliquots of the fractions are subjected to analytical isoelectric focusing to monitor the separation. Figure 4 shows the separation of isoforms 8-11 which may be achieved by this procedure. Isoforms 12-14 which remain bound to the column at the end of the gradient are eluted by washing with a buffer consisting of 10 mM TrisHCl, 140 mM NaCl, 20 mM CuSO₄ (pH 7.0). The isoforms (separated during the gradient or eluted by the sodium chloride solution) are freed of contaminating proteins by reverse phase chromatography followed by gel filtration chromatography as described in Example 2 of Lai et al.

### Example 6: Construction of Human Erythropoietin Analogs

The locations of existing carbohydrate attachment sites within the human erythropoietin amino acid sequence are shown in Figure 5 (SEQ ID. NO: 26). Procedures for generating additional glycosylation sites for erythropoietin are summarized in Figures 6A-C and described below.

The following oligonucleotide primers were synthesized for use in in vitro mutagenesis:

The underlined codons show the mismatched regions where the amino acids indicated in brackets replace the wild-type amino acids.

[Asn⁴, Ser⁶] EPO was constructed to add an N-glycosylation site at Asn 4. [Asn⁹, Ser¹¹] EPO was constructed to add an N-glycosylation site at Asn 9. [Asn⁶⁹] EPO was constructed to add an N-glycosytation site at Asn 69. [Asn¹²⁵, Ser¹²⁷] EPO was constructed to add an N-glycosylation site at Asn 125. [Thr¹²⁵] EPO and [Pro¹²⁴, Thr¹²⁵] EPO were constructed to add an O-glycosylation site at Thr 125.

The following oligonucleotide primers were synthesized for use in in vitro mutagenesis: [Pro¹²⁴, Thr¹²⁵, Thr¹²⁶, Thr¹³¹] EPO: Starting from [Pro¹²⁴, Thr¹²⁵] EPO cDNA, the oligonucleotide primer 5' AGATCCGACCACCGCTGCTCCAC 3' (SEQ ID. NO: 16) is used to generate [Pro¹²⁴, Thr¹²⁵, Thr¹²⁶] EPO. The oligonucleotide primer. 5'TGCTCCACTCACAACAATCACTG 3' (SEQ ID. NO: 17) is then used to generate [Pro¹²⁴, Thr¹²⁵, Thr¹²⁶, Thr¹³¹] EPO.

[Asn⁶⁹, Thr⁷¹] EPO and [Ser⁶⁸, Asn⁶⁹, Thr⁷¹] EPO are constructed to add an N-glycosylation site at Asn 69 and to enhance N-glycosylation at that site. [Asn¹²⁵, Thr¹²⁷] EPO, [Asn¹²⁵, Thr¹²⁷, Thr¹³¹] EPO, [Pro¹²⁴, Asn¹²⁵, Ser¹²⁷] EPO and [Pro¹²⁴, Asn¹²⁵, Thr¹²⁷] EPO are constructed to add an N-glycosylation site at Asn 125 and to increase glycosylation at that site. [Thr¹²⁵, Thr¹²⁶] EPO and [Pro¹²⁴, Thr¹²⁵, Thr¹²⁶, Ser¹³¹] EPO are constructed to add an O-glycosylation site at Thr 125 and to increase glycosylation at that site.

The source of erythropoietin DNA for in vitro mutagenesis was plasmid Hu13, a human erythropoietin cDNA clone in pUC 8 (Law et al. Proc Natl. Acad. Sci. 83, 6920 (1986)). Plasmid DNA derived from Hu13 was digested with BstEII and BgIII restriction enzymes, the resulting DNA fragments were subjected to agarose gel electrophoresis, and the 810 base pair (bp) erythropoietin DNA fragment was isolated from the gel using a GeneClean™ kit and procedures supplied by the manufacturer (BIO 101, Inc.). Plasmid pBRgHuEPO contains the erythropoietin genomic gene as a BamHI fragment inserted into a derivative of pBR322, as described in commonly owned Lin patent, supra. pBRgHuEPO was also digested with BstEll and BgIII and the 6517 bp vector fragment was recovered. Ligation of the two fragments results in IGT1. To construct pEC-1, pDSVL (described in commonly owned Lin patent, supra, and shown in Figure 5B) was digested with BamHI and the isolated 2.8 kilobase (kb) BamHI fragment from IGT1 containing erythropoietin cDNA was ligated into it.

In order to generate single-stranded DNA for in vitro mutagenesis, pEC-1 was digested with BamHI and BglII and the 820 bp erythropoietin cDNA fragment was isolated. It was ligated into the BamHI site of m13mp18 to give m13-EC-1. Single stranded DNA was recovered from supernatants of E. coli strain RZ1032 infected by m13-EC-1 as described by Kunkel et al. Methods in Enzymol. 154, 367 (1987) and Messing, Methods in Enzymol. 101, 20 (1983). For in vitro mutagenesis approximately 1 µg of single-stranded DNA and 0.2 pmole of one of the synthetic primers described above were mixed with 6 µl of buffer (250 mM Tris pH 7.8, 50 mM MgCl_{2,} and 50 mM dithiothreitol). For annealing of the primer to the template, the reaction volume was adjusted to 10 µl with water, the mixture was heated to 65°C for 5 minutes and then allowed to cool to room temperature. For the elongation reaction 2.5 µl of each of dTTP, dATP, dGTP, dCTP and ATP (all at 10 µM) were added, followed by 1 µl (1 unit) of E. coli DNA polymerase (Klenow fragment) and 1 µl (1 unit) of T4 DNA ligase. The mixture was then incubated overnight at 14°C and used to transform E. coli JM 109 (Yanisch-Perron et al. Gene 33, 103 (1985)) as described (Messing, supra).

To identify mutant clones by differential hybridization, plaques on nutrient agar were transferred to Gene Screen filters (New England Nuclear). The filters were dried under a heat lamp and then incubated for one hour in 6x SSC containing 1% SDS at 60°C. For the hybridization, the oligonucleotide primer above (8 pmoles) was end-labeled with T4 polynucleotide kinase and γ ³²P-labeled ATP and incubated with the filters overnight in 6x SSC, 0.5% SDS and 100 mg/ml salmon sperm DNA at 37°C for the [Asn¹²⁴] mutation, 55°C for the [Asn⁴, Ser⁶] mutation, 65°C for the [Thr¹²⁵] and the [Pro¹²⁴, Thr¹²⁵] mutations, and 70°C for the [Asn⁹, Ser¹¹] and [Asn¹⁶³, Ser¹⁶⁵] mutations. The next day, the filters were washed three times with 6x SSC at room temperature and subjected to autoradiography. If necessary, the filters were then washed with 6x SSC at increasing temperatures until little or no hybridization was detected to plaques having the wild-type erythropoietin cDNA sequence. Clones that gave positive hybridization signals under these conditions were identified and retransfected into JM109 to isolate a pure clone. Dideoxy chain termination sequence analysis indicated that the mutations to asparagine, serine threonine and proline residues were present.

Double stranded m13 EC-1 DNAs carrying the [Asn⁴, Ser⁶], [Asn⁹, Ser¹¹], [Asn⁶⁹], [Asn¹²⁴], [Asn¹²⁵], [Ser¹²⁷], [Asn¹⁶³, Ser¹⁶⁵] [Thr¹²⁵], and [Pro¹²⁴, Thr¹²⁵] changes were recovered from JM109 transfected cells by the boiling method (Holmes et al. Anal. Biochem 117, 193 (1981)). The DNAswere digested with BstEll and Xholl and the 810 bp erythropoietin DNA fragments were isolated. pEC-1 was digested with BstEll followed by a partial digestion with BgIII and the 5' termini of the resulting fragments are dephosphorylated with bacterial alkaline phosphatase in 10 mM Tris, pH 8 at 60°C for 60 minutes. The 7 kb vector fragment lacking the 810 bp BstEII-BglII fragment was isolated and ligated to the erythropoietin fragments above. The resulting plasmids (designated pEC-X where X is the analog number) contain DNA encoding erythropoietin analogs having altered amino acid residues at the indicated positions.

Alternatively, an analog of erythropoietin (pEC34) was constructed by in vitro mutagenesis that deleted amino acid residues 41-55. This resulted in a smaller (775 bp) EPO containing BstEII-Bg1II fragment. The fragment was inserted into pEC1 as described above. For cloning analogs of erythropoietin, pEC34 was digested with BstEII, partially digested with Bg1II, dephosphorylated and the vector isolated as described above. The 7kb vector fragment was then ligated to erythropoietin fragments as described above. Cloning with pEC34 allows easy discrimination between recombinants and simple reclosures. Reclosures result in a smaller BstEII-Bg1II fragment than analogs and these can be easily differentiated on agarose gels.

These general procedures were used to construct the erythropoietin analogs shown in Tables 3, 4 and 5. The DNA sequence changes for each of the analogs are shown; otherwise the oligonucleotide primers used for mutagenesis had sequences complimentary to those of human erythropoietin.

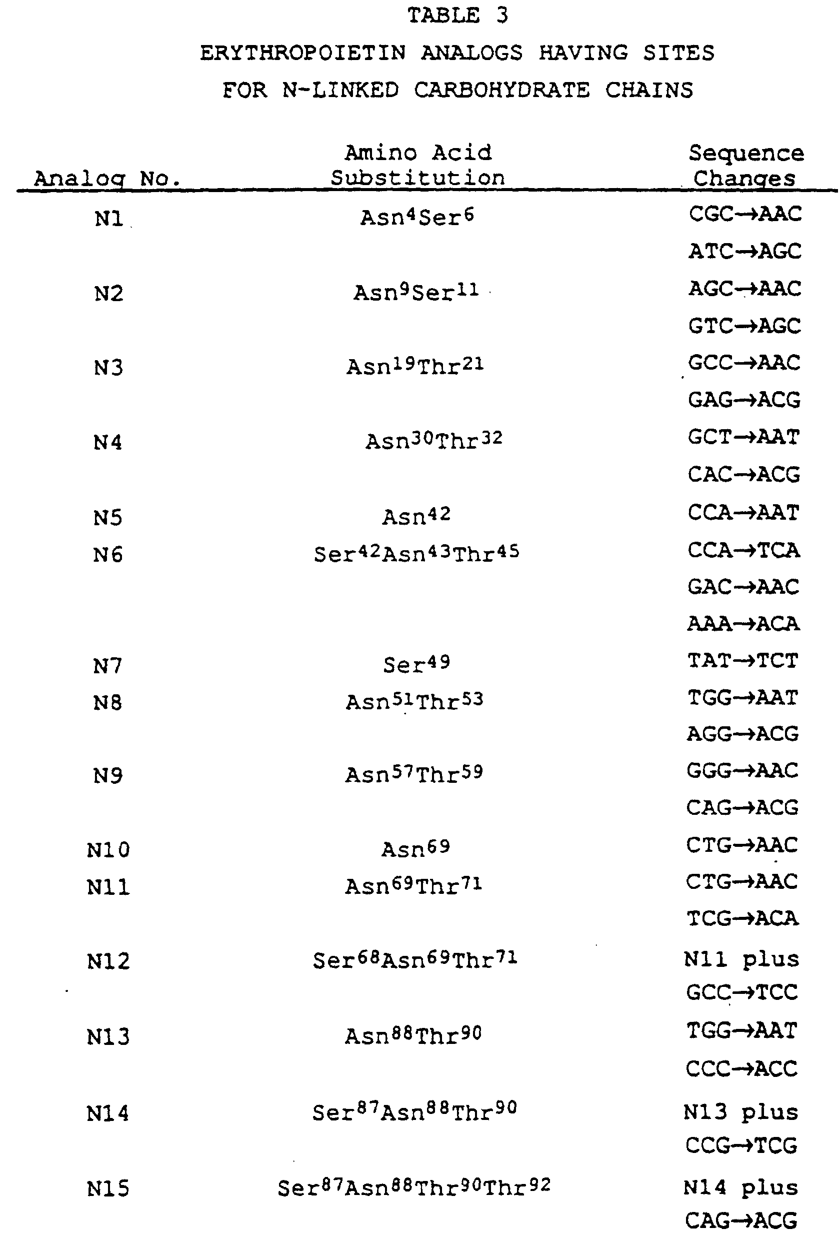

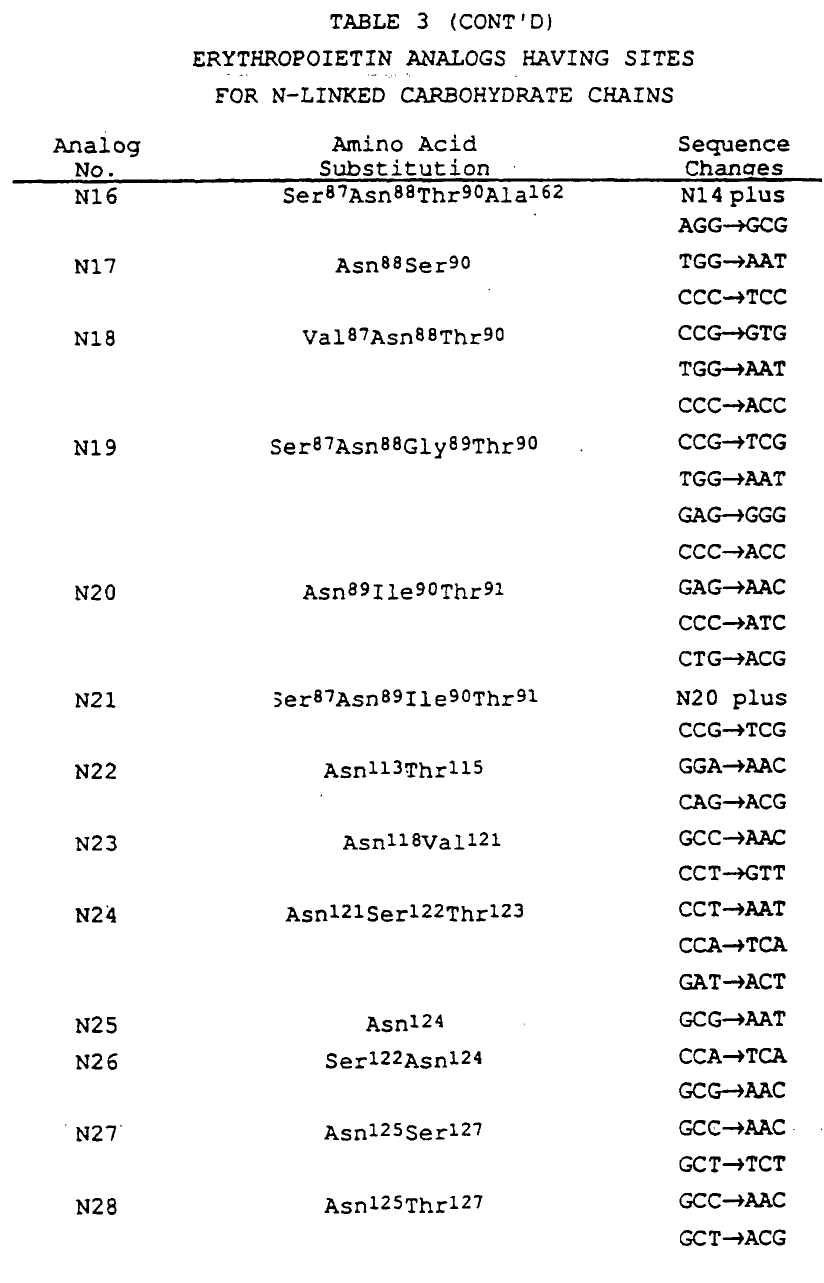

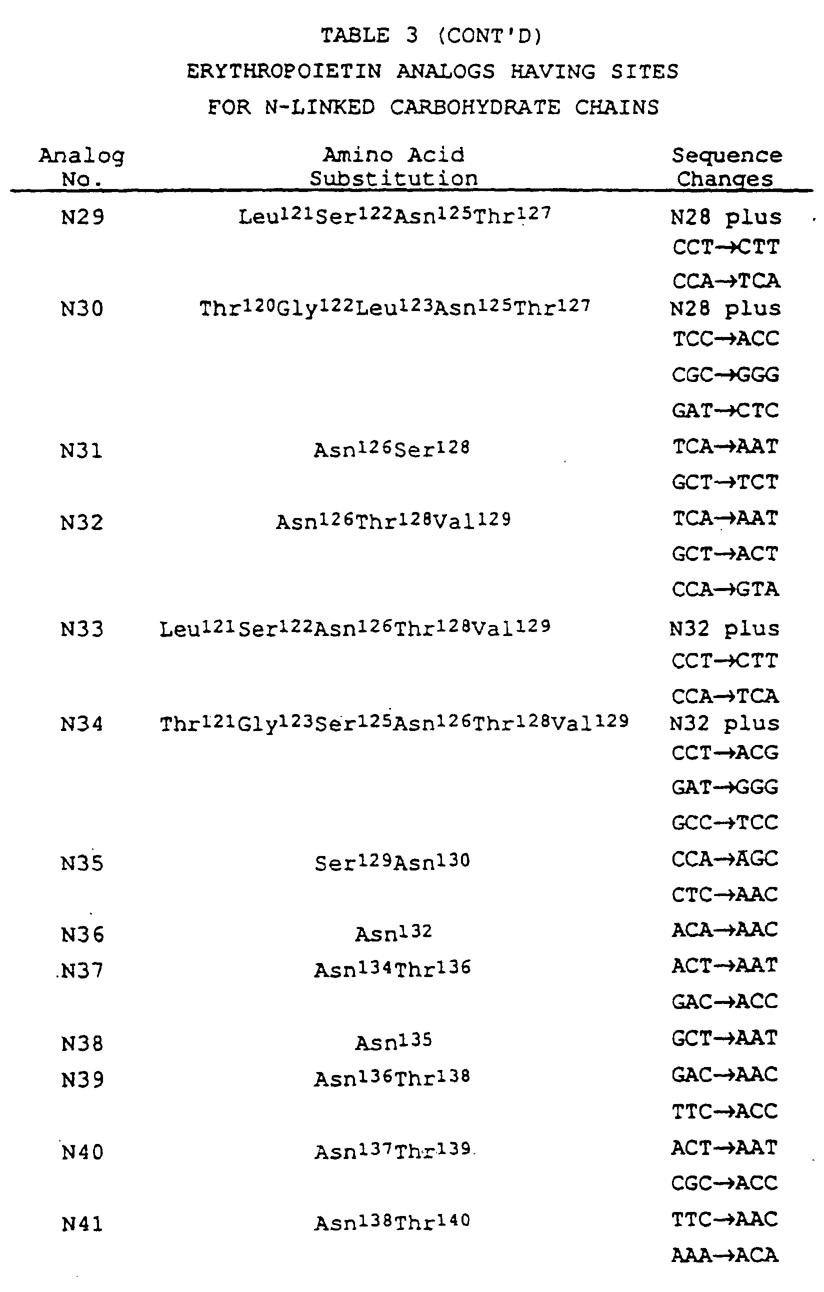

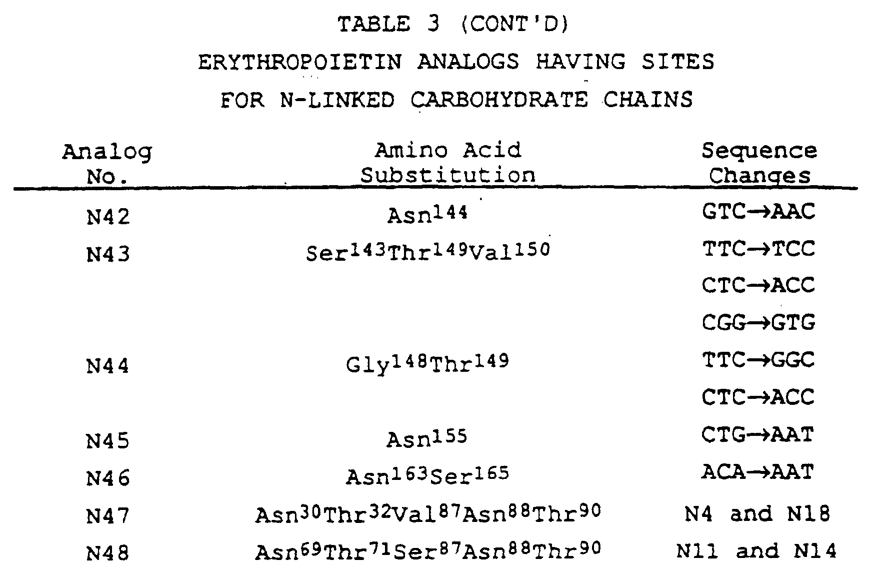

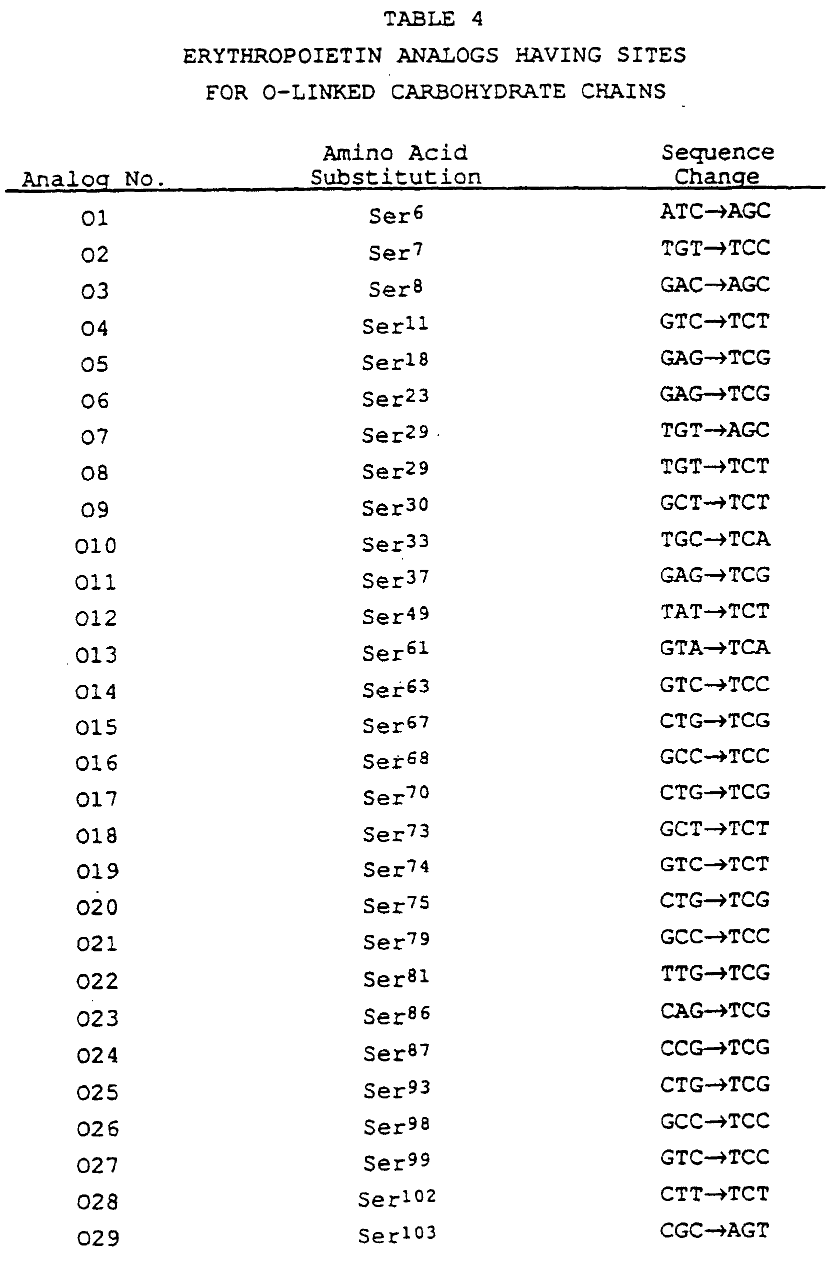

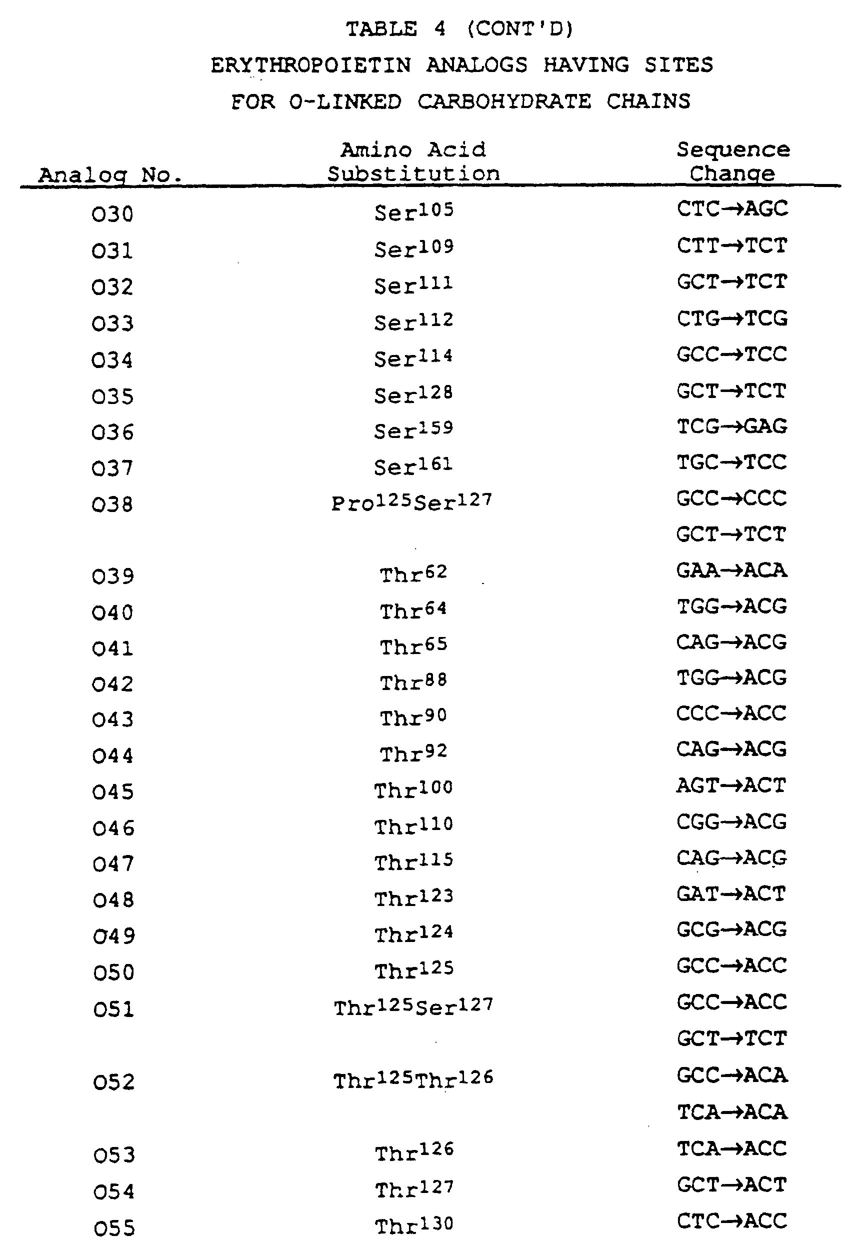

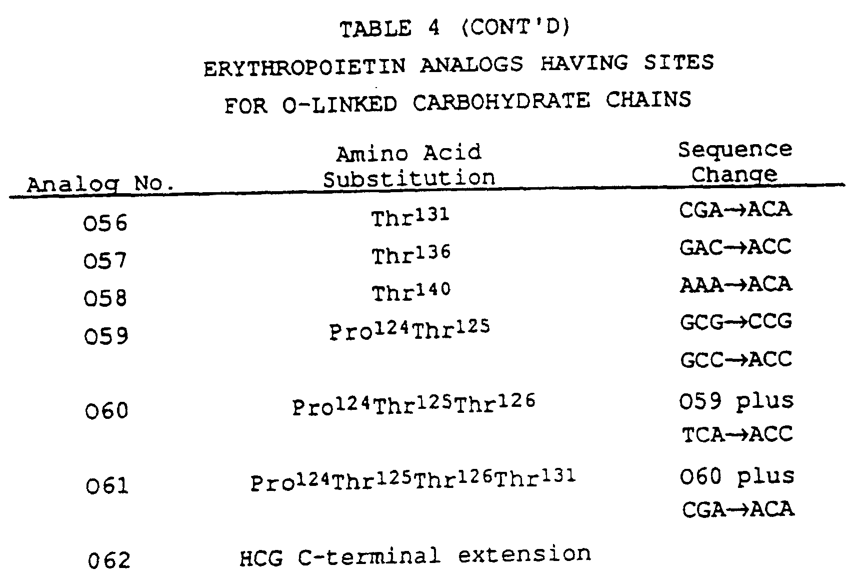

**TABLE 5**

| ERYHROPOIETIN ANALOGS HAVING SITES FOR N- AND O-LINKED CARBOHYDRATE CHAINS | | |
|---|---|---|
| Analog No. | Amino Acid Substitution | Sequence Change |
| NO1 | Ser⁸⁷Asn⁸⁸Thr⁹⁰ HCG C-terminal extension | N14 and O62 |
| NO2 | Asn³⁰Thr³²Val⁸⁷Asn⁸⁸Thr⁹⁰ HCG C-terminal extension | N47 and O62 |

Plasmids designated pDEC-X (where X is the analog number) were constructed by inserting erythropoietin cDNA into pDECΔ, which is a derivative of plasmid pDSα2. The expression vector pDSα2 is generally described in PCT Application No. WO 90/14363. pDECΔ was derived from pDSα2 by the following steps:
(1) The HindIII site of pDSα2 was deleted by digesting pDSα2 DNA with HindIII, treating the HindIII cohesive ends with E. coli DNA Polymerase (Klenow fragment) and dNTPs, and religating the blunt-ended vector. The resulting plasmid was pDSα2ΔH.
(2) pDSα2ΔH was digested with Sall and a synthetic oligonucleotide having an SV40 splice signal with a Sall linker attached to the 3' end of the splice signal was ligated to it. The synthetic oligonucleotide had the following sequence (SEQ ID. NO: 18): The resulting plasmid was pDSα2ΔH splice.
3) pDSα2ΔH splice was digested with Sall and blunt-ended by treating the cohesive ends with T4 DNA polymerase and dNTPs. An 820 bp. BamHI-BglII human erythropoietin cDNA fragment was blunt-ended by the same method and ligated to the plasmid. The resulting plasmid was pDEC-1.
4) pDEC was digested with Kpnl and Pvull and blunt-ended by treating the cohesive ends with mung bean nuclease. The plasmid was religated to delete the excised Kpnl-Pvull fragment resulting in the plasmid pDECΔ.

pDEC-X plasmids were made from pDECΔ by complete digestion with BstEII followed by a partial digestion with Bg1II. The vector fragment lacking erythropoietin coding sequences was isolated and ligated to the 810 bp BstEII-Bg1II fragments containing the desired plasmid.

Details of the construction of some analogs having multiple amino acid changes are described below.

### Construction of pDEC(N47) and pDEC(N48)

pDEC(N47) which contains asn30 thr32 val87 asn68 and thr90 mutations was constructed from pDEC(N18) and pDEC(N4). pDEC(N18) was digested with HindII and Bg1II and a 445 bp fragment was isolated. pDEC(N4) was digested with BstEII and HindIII and a 377 bp fragment was isolated. These two fragments were ligated into pDECΔ cut with BstEII and Bg1II as described above resulting in pDEC(N47).

pDEC(N48) which contains asn69 thr71 ser87 asn88 and thr90 mutations was constructed from pDEC(N14) and pDEC(N11). pDEC(N14) was digested with HindII and Bg1II and a 445 bp fragment was isolated. pDEC(N11) was digested with BstEII and Hindll and a 377 bp fragment was isolated. These two fragments were ligated into pDECΔ cut with BstEII and Bg1II as described above resulting in pDEC(N48).

### Construction of pDEC(O62) (HCG-Erythropoietin Fusion)

pDEC(O62) was assembled from pEC1 and a 107 base pair Stul-BgIII synthetic DNA linker containing the carboxy terminal 28 amino acids from human chorionic gonadotropin (ser-ser-ser-ser-lys-ala-pro-pro-pro-ser-leu-pro-ser-pro-ser-arg-leu-pro-gly-pro-ser-asp-thr-pro-ile-leu-pro-gln) (SEQ ID. NO: 25) (Pierce et al. Ann. Rev. Biochem. 50, 465 (1981)). The sequence of the linker is as follows:

pEC1 was digested with Stul and BgIII and the 610bp DNA fragment was isolated. The synthetic linker was phosphorylated with ATP and polynucleotide kinase and ligated with the pEC1 fragment into pDECΔ previously digested with BstEII and partially digested with BglII as described above.

### Construction of pDEC(NO1)

pDEC(NO1) was assembled from pDEC(O62) (HCG-EPO) and pDEC(N14) (Ser⁸⁷Asn⁸⁸Thr⁹⁰). pDEC177 was digested with Stul and BgIII and the 610bp DNA fragment containing the Ser⁸⁷Asn⁸⁸Thr⁹⁰ mutations was isolated with gene-clean. pDEC(O62) was digested with Stul and BgIII and the 107 base pair fragment was isolated. These two DNA fragments were ligated into pDECΔ previously digested with BstEII and partially digested with BgIII as described above.

### Construction of pDEC(NO2)

pDEC(NO2) was assembled from pDEC(O62) (HCG-EPO) and pDEC(N47) (Asn³⁰Thr³²Val⁸⁷Asn⁸⁸Thr⁹⁰). pDEC(N47) was digested with Stul and BgIII and the 610bp DNA fragment containing the Asn³⁰Thr³²Val⁸⁷Asn⁸⁸Thr⁹⁰ mutations was isolated with GeneClean™. pDEC(O62) was digested with Stul and BgIII and the 107 base pair fragment was isolated. These two DNA fragments were ligated into pDECΔ previously digested with BstEll and partially digested with BgIII as described above.

### Construction of pDEC(N16) (Ser⁸⁷Asn⁸⁸Thr⁹⁰Ala¹⁶²).

pDEC(N16) was assembled from pDEC(N14)(Ser⁸⁷Asn⁸⁸Thr⁹⁰) and pDEC258(Ala¹⁶²). pDEC258 was constructed using procedures for in vitro mutagenesis described above and changing the AGG codon to GCG at position 162. pDEC(N14) was digested with Stul and BgIII and the 610bp DNA fragment containing the Ser⁸⁷Asn⁸⁸Thr⁹⁰ mutations was isolated with GeneClean™. pDEC258 was digested with Stul and BgIII and a 210 base pair fragment was isolated. These two DNA fragments were ligated into pDECΔ previously digested with BstEII and partially digested with BgIII as described above.

### Construction of pDEC(R1), (R2) and (R3)

To remove glycosylation sites from pDEC(N14), m13-EPO(N14) containing ser87 asn88 and thr90 mutations was subjected to in vitro mutagenesis as described above using the following primers: The resulting plasmids were designated pDEC(R1) (gln²⁴ ser⁸⁷ asn⁸⁸ thr⁹⁰) pDEC(R2) (gln³⁸ ser⁸⁷ asn⁸⁸ thr⁹⁰) and pDEC(R3) (gln⁸³ ser⁸⁷ asn⁸⁸ thr⁹⁰). m13EC-1 was also subjected to in vitro mutagenesis with the above oligonucleotide primers resulting in pEC10 (gln²⁴) and pEC8 (gln³⁸). pEC9 (gln⁸³) was constructed using the primer

cDNA clones of human erythropoietin and analogs corresponding to [Asn⁴, Ser⁶] EPO, [Asn⁹, Ser¹¹] EPO, [Asn⁶⁹] EPO, [Asn¹²⁴] EPO, [Asn¹²⁵, Ser¹²⁷] EPO, [Asn¹⁶³, Ser¹⁶⁵] EPO, [Thr¹²⁵] EPO and [Pro¹²⁴, Thr¹²⁵] EPO and cDNA clones of analogs described in Tables 3, 4 and 5, were transferred into COS-1 cells (ATCC No. CRL-1650) by electroporation. COS-1 cells were harvested from semi-confluent dishes, washed with medium (Dulbecco's modified essential medium containing 5% fetal calf serum and 1% L-glutamine/penicillin/streptomycin (Irvine Scientific)) and resuspended at 4 x 10⁶ cells/ml. One ml of cells was transferred to an electroporation cuvette (Bio-Rad) and electroporated with a Bio-Rad Gene Pulser at 25 µFarads and 1600 volts in the presence of 100 to 200 µg of carrier DNA and 2 to 20 µg of plasmid DNA encoding the erythropoietin analog. The electroporated cells were plated at 2 x 10⁶ cells per 60 mm tissue culture dish in 5 ml of medium. Two to four hours after plating the medium was replaced with 5 ml of fresh medium. The conditioned medium was collected 3 to 5 days after electroporation.

### Example 7: Characterization of Erythropoietin Analogs

### A. Determination of Carbohydrate Addition

A volume of supernatant containing 5-20 units from COS cells transfected with erythropoietin analog cDNAs as described in Example 6 was immunoprecipitated overnight at room temperature with a rabbit anti-erythropoietin polyclonal antibody. 20-80 µl of 1:1 Protein A-Sepharose in phosphate buffered saline (PBS) was added to the immunoprecipitate and allowed to incubate for one hour at room temperature. The samples were centrifuged, washed with PBS and, where indicated, the pellet was treated with N-glycanase to remove N-linked carbohydrate chains. The samples were analyzed by 15% SDS-polyacrylamide gel electrophoresis, transferred to nitrocellulose and subjected to Western analysis as described (Burnette et al. Anal. Biochem. 112, 195-203 (1981); Elliott et al. Gene 79, 167-180 (1989)) using a mixture of mouse anti-erythropoietin monoclonal antibodies. One such antibody, 9G8A, is described in Elliott et al. (1989) Blood 74, Supp. 1, A. 1228.

Analysis of COS cell supernatants transfected with [Asn⁶⁹] EPO and [Asn¹²⁵, Ser¹²⁷] EPO cDNA revealed increased protein size compared to human sequence erythropoietin. This increased size is indicative of an additional N-linked carbohydrate chain (Figure 7). Treatment of supernatants from COS cells transfected with [Thr¹²⁵] EPO and [Pro¹²⁴, Thr¹²⁵] EPO cDNA with N-glycanase revealed an increased protein size compared to human sequence erythropoietin. This increased size is indicative of an additional O-linked carbohydrate chain (Figure 8). Western blot analysis of other selected analogs is shown in Figure 10.

To determine the number of N-linked carbohydrate chains attached to EPO, partial N-glycanase digestions were performed. Analogs or rHuEPO were expressed in CHO cells and conditioned serum free medium was collected. Tubes contained 40 units of EPO (volume adjusted to 15 µl with H₂O) To each tube was added 10 µl 0.5% SDS and each sample was boiled for 3 minutes. Then the following components were added, 10.8 µl 0.5M NaPO₄ pH8.6, 5µl 7.5% nonidet P40 and 1.5 µl of 250 unit/ml N-glycanase (Genzyme). Samples were incubated for the indicated times at 37°C. The reaction was stopped by the addition of SDS-PAGE sample buffer (see above) and then subjected to SDS-PAGE western analysis (10% acrylamide) using an anti-EPO polyclonal antibody and a anti-rabbit Vectastain™ kit (Vector laboratories) with 4-chloronaphthol as substrate. An analysis of N-linked chains using this method is shown in Figure 11 for human erythropoietin and analog N14.

### B. Assays for erythropoietin analog activity

RIAs were performed according to Egrie et al., supra. EIAs were performed with CLINIGEN™ EIA kit (R and D Systems) using procedures supplied by the manufacturer. The in vivo biological activity of erythropoietin analogs was determined on supernatants from CHO cells expressing an erythropoietin analog or on purified erythropoietin obtained from CHO cell conditioned medium as described below using the exhypoxic polycythemic mouse bioassay (Cotes et al., supra).

In vitro erythropoietin activity was determined by the erythroid colony forming assay as described by Iscove et al. J. Cell Physiol. 83, 309-320 (1974) with modifications. The mononucleated cells from human bone marrow cells were partially purified on a ficoll-paque cushion and washed in Iscove medium before plating to remove the adherent cells. The culture medium contained 0.9% methyl cellulose and did not include any bovine serum albumin. The erythroid colonies were scored after 8 to 10 days of culture.

The erythropoietin analogs transfected and expressed in COS cells as described in Example 6 were analyzed in crude COS cell supernatants using RIA, EIA and an erythroid colony forming assay. Purified human sequence erythropoietin has an in vitro activity that was comparable to the RIA activity as determined by the above-mentioned assays. The analogs [Asn⁶⁹] EPO, (Thr¹²⁵) EPO and [Pro¹²⁴, Thr¹²⁵] EPO exhibited an in vitro activity that was comparable to the RIA activity and gave evidence of having additional carbohydrate chains (as determined in Section A). [Thr¹²⁵] EPO and analogs N4, N11, N14, N16, N18, N47, N48, O62, NO1 and NO2 were analyzed further by transfecting a cDNA clone encoding the erythropoietin analog into CHO cells, and subjecting the CHO cell supernatants to RIA or EIA and in vivo biological assays. The results are shown in Table 6. In vivo activities for analogs R1, R2 and R3 expressed in CHO cell supernatants are shown in Table 7.

**TABLE 6**

| ERYTHROPOIETIN ANALOGS HAVING ADDITIONAL CARBOHYDRATE CHAINS | | | |
|---|---|---|---|
| EPO Sequence | N-Linked^{a} Chains | O-Linked Chains | *In Vivo*^{b} Activity RIA or EIA |
| Human | 3^{c} | 1^{d} | 0.6 |
| Asn³⁰Thr³² | 3 to 4^{c} | n.t. | 1.1 |
| Asn⁵¹Thr⁵³ | 4 | n.t. | n.t. |
| Asn⁵⁷Thr⁵⁹ | 4 | n.t. | n.t. |
| Asn⁶⁹ | 4 | reduced^{e} | n.t. |
| Asn⁶⁹Thr⁷¹ | 4 | n.t. | 0.25-0.7 |
| Ser⁶⁸Asn⁶⁹Thr⁷¹ | 4 | n.t. | n.t. |
| Val⁸⁷Asn⁸⁸Thr⁹⁰ | 4^{c} | n.t. | 1.8 |
| Ser⁸⁷Asn⁸⁸Thr⁹⁰ | 3 to 4^{c} | normal | 1.0 |
| Ser⁸⁷Asn⁸⁸Gly⁸⁹Thr⁹⁰ | 4 | n.t. | n.t. |
| Ser⁸⁷Asn⁸⁸Thr⁹⁰Thr⁹² | 4 | reduced^{e} | n.t. |
| Asn⁸⁹Ile⁹⁰Thr⁹¹ | 4 | n.t. | n.t. |
| Ser⁸⁷Asn⁸⁹Ile⁹⁰Thr⁹¹ | 4 | n.t. | n.t. |
| Ser⁸⁷Asn⁸⁸Thr⁹⁰Ala¹⁶² | 4 | n.t. | 1.8 |
| Asn¹³⁶Thr¹³⁸ | 3 to 4 | n.t. | n.t. |
| Asn¹³⁸Thr¹⁴⁰ | 3 to 4 | n.t. | n.t. |
| Asn⁶⁹Thr⁷¹Ser⁸⁷Asn⁸⁸Thr⁹⁰ | 4 to 5 | n.t. | 0.025-0.25 |
| Asn³⁰Thr³²Val⁸⁷Asn⁸⁸Thr⁹⁰ | 4 to 5^{c} | normal | 1.8 |
| Thr¹²³ | 3 | 1 and 2^{f} | n.t. |
| Thr¹²⁵ | 3 | 1 and 2^{f} | 0.7 |
| Pro¹²⁴Thr¹²⁵ | 3 | 1 and 2^{f} | n.t. |
| HCG C-terminal extension | 3 | at least 3^{g} | 1.0-1.3 |
| | | | |
| Ser⁸⁷Asn⁸⁸Thr⁹⁰ HCG extension | 4 | at least 3^{g} | 1.5 |
| | | | |
| Asn³⁰Thr³²Val⁸⁷Asn⁸⁸Thr⁹⁰ HCG extension | 4 to 5 | at least 3^{g} | 0.8 |

| | | | |
|---|---|---|---|
| ^{a}The number of additional N-linked chains was estimated based upon the mobility of the analog polypeptides in SDS gels as described in Example 7A. | | | |
| bRatio of in vivo activity of analog to amount of erythropoietin analog. Activity measurements were done on CHO cell supernatants of analogs using the mouse polycythemic bioassay. Quantities of erythropoietin analogs in CHO cell supernatants were determined by RIA or EIA as described in the text. | | | |
| ^{c}Confirmation of the number of additional carbohydrate chains was made by examining the glycoprotein migration in SDS-gels after partial N-glycanase digestion as described in Example 7A. | | | |
| ^{d}O-linked chain at Ser¹²⁶ is present on 70% of human erythropoietin molecules. | | | |
| ^{e}Analogs having reduced O-glycosylation have a carbohydrate chain at Ser¹²⁶ on less than 70% of the molecules. | | | |
| ^{f}Thr¹²³ EPO has two O-linked chains on greater than 60% of the molecules. Thr¹²⁵ EPO has two O-linked chains on about 40% of the molecules. Pro¹²⁴Thr¹²⁵ EPO has two O-linked chains on greater than 80% of the molecules. | | | |
| ^{g}These analogs have at least three O-linked chains and may have four or five. HCG alone is known to have four O-linked chains. N.T. NOT TESTED | | | |

**TABLE 7**

| ACTIVITY OF HUMAN ERYTHROPOIETIN AND ANALOGS HAVING CARBOHYDRATE SITE REARRANGEMENTS | | |
|---|---|---|
| EPO Sequence | N-Linked Chains | *In vivo* Activity RIA or EIA |
| Human | 3 | 0.69 |
| Gln²⁴ | 2 | 0.16 |
| Gln³⁸ | 2 | 0.16 |
| Gln⁸³ | 2 | 0.23 |
| Gln²⁴Ser⁸⁷Asn⁸⁸Thr^{90 (R1)} | 3 | 0.69 |
| Gln³⁸Ser⁸⁷Asn⁸⁸Thr^{90 (R2)} | 3 | 0.41 |
| Gln⁸³Ser⁸⁷Asn⁸⁸Thr^{90 (R3)} | 3 | 0.50 |
| Ser⁸⁷Asn⁸⁸Thr⁹⁰ | 3 to 4 | 1.48 |
| Ratio of in vivo activity to RIA or EIA was determined as described in footnote a of Table 6. | | |

### C. Preparation of Isoform Mixtures Derived from Erythropoietin Analogs

### [Thr¹²⁵] EPO (EPO O50)

The erythropoietin analog [Thr¹²⁵] EPO was constructed as described in [Section A] Example 6. An 810 bp. erythropoietin cDNA fragment carrying the [Thr¹²⁵] mutation was isolated by cleaving the plasmid pEC containing the [Thr¹²⁵] mutation with BstEll and BgIII and ligating the fragment to pDECΔ, [a derivative of pDSα2] (described in Example 6).

Plasmid pDECΔ containing [Thr¹²⁵] erythropoietin cDNA was transfected into DHFR-deficient CHO cells. 770 ml of CHO cell conditioned medium was concentrated using a 10,000 dalton molecular weight cut-off membrane and diafiltered against 10 mM Tris-HCl, pH 8.6 to a final volume of 34 ml. A 17 ml. aliquot of the concentrate was loaded onto a Q-Sepharose fast flow column (5 ml bed volume) equilibrated in the same buffer and eluted in a linear gradient of 0-250 mM NaCl in 10 mM Tris-HCl, pH 8.6. Aliquots of column fractions, either untreated or digested with N-glycanase, were analyzed by SDS-PAGE or IEF and pools (designated 2, 3 and 4) were made based upon the isoform and/or carbohydrate composition of the fractions. Each pool was loaded onto a Vydac C4 column (214TPB 2030; 1 cm diameter; 1.8-2.5 ml bed volume; 0.34 ml/min) and washed with two column volumes of 20% ethanol in 10 mM Tris-HCl, pH 7.0. The columns were eluted with linear gradients of 20-94% ethanol, 10 mM Tris, pH 7.0. Pools were made, diluted into 10 mM Tris-HCl, pH 7.0, and loaded onto Q-Sepharose fast flow columns. Following a wash in 10 mM Tris-HCl, pH 7.0, the samples were eluted with 20 mM sodium citrate, 250 mM NaCl, pH 7.0. The purified [Thr¹²⁵] pools were analyzed by IEF and are shown in Fig. 9. These pools were also analyzed for in vivo biological activity as described above (Cotes et al., supra) and the results shown in Table 8.

### Ser⁸⁷Asn⁸⁸Thr⁹⁰ EPO (EPO N14)

### Prep. 1

The EPO N14 analog was purified using a three step procedure consisting of ion exchange chromatography, reverse phase chromatography and gel filtration chromatography. During the ion exchange step, fractions were pooled to give a mixture of isoforms having high levels of sialic acid. Two additional pools were made during reverse phase chromatography containing the analog with or without aggregate. The details of the purification are set out below.
1. Conditional medium from CHO cells expressing the EPO N14 analog was harvested and concentrated approximately 2.5-fold using a stirred cell with YM10 membrane (Amicon) and diafiltered against 10mM Tris pH 8.5.
2. The concentrated medium was loaded at ∼ 12 A₂₈₀/ml resin to Q-Sepharose FF (Pharmacia) column equilibrated in 10mM Tris pH 8.5 at a flow rate of 0.2 cm/min.
3. Following the load, the column was washed with 3 column volumes (CV) of 10mM Tris pH 8.5 and eluted with a gradient of 0-0.5M NaCl/10mM Tris pH 8.5 in 50CV. 1 CV fractions were collected.
4. A sample of each fraction was run on an IEF gel pH 3-5. Based on the isoform distribution on IEF, a fraction pool containing largely isoforms 11-18 was made. EDTA was added to the pool to 1mM final concentration.
5. The isoform pool was loaded at ∼5 A₂₈₀/ml resin to a reverse phase C4 column (Vydac) equilibrated in 20% ethanol/10mM Tris pH 7.0 at a flow rate of 2.5 cm/min. The column was washed with 1 CV of 20% ethanol/10mM Tris pH 7.0 and eluted with a gradient of 20-94% ethanol/10mM Tris pH 7.0 in 30CV at a flow rate of 1 cm/min. 0.2CV fractions were collected.
6. A sample of each fraction was analyzed by non-reducing 12% SDS-PAGE. Two separate pools were made based on the presence of aggregate observed on SDS gels. Pool #1 contained EPO analog but no aggregate. Pool #2 contained both EPO analog and aggregate.
7. Pool #1 was concentrated approximately 65-fold and pool #2 approximately 250-fold using Centricon 10's (Amicon). Each pool was buffer exchanged into 20mM NaCitrate/100mM NaCl pH7.0.
8. Each pool was individually purified on HPLC BioSil SEC-250 (BioRad) column equilibrated in 20mM NaCitrate/100mM NaCl pH7.0. Pools were loaded at <6 A280/ml resin at a flow rate of 2.26 cm/min. Peaks corresponding to monomeric analogs were collected from each run.
9. The absorbance of each pool was measured and a portion of each pool was concentrated for analysis by SDS-PAGE and IEF gels. Pool #1 had a distribution of isoforms 15-17 and was used for pharmacokinetic and receptor binding studies.

### Prep. 2

EPO N14 analog was purified using a three step procedure consisting of ion exchange chromatography, reverse phase HPLC, and hydroxylapatite chromatography. During the ion exchange step, the analog was divided into three pools containing different mixtures of isoforms. The details of the purification are set out below.
1. Supernatant from CHO cells expressing EPO N14 were harvested and concentrated approximately 10-fold using a Filtron Mini-Ultrasette tangential flow device and diafiltered against 10mM Tris pH 8.5.
2. The concentrated medium was loaded at ∼ 10 A₂₈₀/ml resin to Q-Sepharose FF (Pharmacia) column equilibrated in 10mM Tris pH 8.5 at a flow rate of 0.2 cm/min.
3. Following the load, the column was washed with three column volumes (CV) of 10mM Tris pH8.5 and eluted with a gradient of 0-0.5M NaCl/10mM Tris pH 8.5 in 50CV. 1 CV fractions were collected.
4. A sample of each fraction was run on an IEF gel pH 3-5. Three separate fraction pools were prepared based on the isoform distribution as determined by IEF. A low isoform pool (isoform 4-12), a medium isoform pool (isoform 5-15), and a high isoform pool (isoform 6-18) were made.
5. Each isoform pool was individually purified on a Vydac C4 reverse phase HPLC column. The column was developed with a gradient from 0.1% TFA/H₂0 to 0.1% TFA/75% acetonitrile at a rate of 1% increase in acetonitrile per minute.
6. The analog peak from each pool was collected and diluted with 4 volumes 80mM Tris HC1/20mM Tris base, then concentrated and buffer exchanged into 10mM Tris pH 7.2 using solvent resistant Centricon 3's.
7. Each sample was diluted to 2 A₂₈₀/ml in 10mM Tris pH 7.2 and an equal volume of 4M Guanidine HC1 (GuHC1), 10mM CHAPS, 10mM Tris pH 7.2 was added for a final sample concentration of 1 A₂₈₀/ml. Each sample was loaded onto a hydroxylapatite minicolumn equilibrated with 2M GuHC1, 5mM CHAPS, 10mM Tris pH 7.2. The column was washed with equilibration buffer and 1 CV fractions of flow through were collected.
8. The absorbance of fractions was measured and pools were made and buffer exchanged into 10mM Tris pH 7.2 using Centricon 10's. The absorbance of each pool was measured.

The final pools were analyzed by SDS-PAGE and IEF gels. The IEF gels are shown in Figure 12. The RIA and in vivo activity assays were performed as described in Example 7A. The in vivo activity of the final isoform pools is reported in Table 8. The high sialic acid isoform pool was used in experiments to determine the increase in the hematocrit of mice.

**TABLE 8**

| ACTIVITY OF ISOFORMS FROM ERYTHROPOIETIN ANALOGS | | |
|---|---|---|
| EPO Sequence | Isoform Pool | *In vivo* activity u/mg. peptide^{a} |
| Thr¹²⁵ | Isoforms 8-12 | 147,000^{b} |
| | Isoforms 9-15 | 215,000^{b} |
| | Isoforms 13-15 | 111,000^{b} |
| Ser⁸⁷Asn⁸⁸Thr⁹⁰ | Isoforms 7-12 | 132,000 ± 17,000 |
| | Isoforms 10-14 | 233,000 ± 39,000 |
| | Isoforms 12-17 | 272,000 ± 14,000 |

| | | |
|---|---|---|
| ^{a}milligrams of erythropoietin peptide were calculated from A₂₈₀ of the Ser⁸⁷Asn⁸⁸Thr⁹⁰ EPO isoform pools using an extinction coefficient of 0.93 for a 1mg/ml solution. | | |
| ^{b}Standard deviations for Thr¹²⁵ EPO isoform pools are not reported since activity assays were done either once or twice. | | |

### Example 8: Biological Properties of EPO N14 Analog

The activities of isoform pools of EPO N14 analog, recombinant human erythropoietin (rHuEPO) and isolated rHuEPO isoform 14 were compared in i.v. pharmacokinetic assays, receptor binding assays and hematocrit studies. EPO N14 isoform pools were prepared as described in Example 7C. rHuEPO was prepared according to Lai et al. supra. rHuEPO isoform 14 was purified as follows: rHuEPO consisting of a mixture of isoforms 10, 11, 12, 13, 14 and 15 was loaded to a Q-Sepharose Fast Flow column (Dimensions = 2.2 cm dia. X 3.4 cm ht.) equilibrated in 10 mM Tris pH 7.2. The loaded column was equilibrated to 2mM acetic acid / 6 M urea ("A" buffer) then a multiphasic gradient designed to optimize purification of isoforms 13 and 14 was run. The gradient was 0% to 7.3% 800 mM acetic acid / 6 M urea ("B" buffer) in 750 mL, 7.3% to 11.4% "B" buffer in 750 mL, 11.4% to 26.8% "B" buffer in 1250 mL, 26.8% to 62.4% "B" buffer in 1250 mL, then 62.4% to 100% "B" buffer in 700 mL. Fractions of 12.5 mL were collected, neutralized with ammonium hydroxide, then assayed by isoelectric focusing in polyacrylamide gels. Those fractions containing pure isoform 14 were pooled together, concentrated with an Amicon stirred cell equipped with a YM-10 membrane then buffer exchange to water.

### A. IV Pharmacokinetics

Two separate studies were performed to compare the pharmacokinetic parameters of EPO N14 analog (isoforms 15-17) and isolated isoform 14 to rHuEPO.

In each study 1 µCi of either ¹²⁵I-isolated isoform 14, ¹²⁵I-EPO N14 analog, or ¹²⁵I-recombinant human erythropoietin (Amersham) was injected intravenously into a carotid cannula in male Sprague-Dawley rats weighing between 310-378g. At various time points after administration, 0.3ml of blood was collected and serum was prepared by centrifugation. The ¹²⁵I-EPO (either recombinant human, isolated isoform 14, or EPO N14 analog) concentration in 0.1ml of each serum sample was then determined following an overnight 4°C incubation with 90% ethanol. The ethanol-precipitated ¹²⁵I-EPO in each serum sample was counted in a gamma counter and the resulting pharmacokinetic curves are shown in figure 13. The pharmacokinetic parameters were determined for each rat using PCNONLIN 4.0 nonlinear regression analysis (Statistical Consultants, 1992) and the results for each group were averaged. The results from the EPO N14 analog and the isolated isoform 14 pharmacokinetic studies are summarized in Table 9.

As seen in Table 9, there is a significant difference in the serum clearance of EPO N14 analog when compared to that calculated for recombinant human erythropoietin. The recombinant human erythropoietin displayed the fastest beta half-life of 3.10 hrs compared to 3.97 hrs for isolated isoform 14 and 4.36 hrs for EPO N14 analog. The recombinant human erythropoietin group also had the fastest clearance half-life of 1.78 hrs compared to 2.40 hrs for isolated isoform 14 and 3.03 hrs for EPO N14 analog.

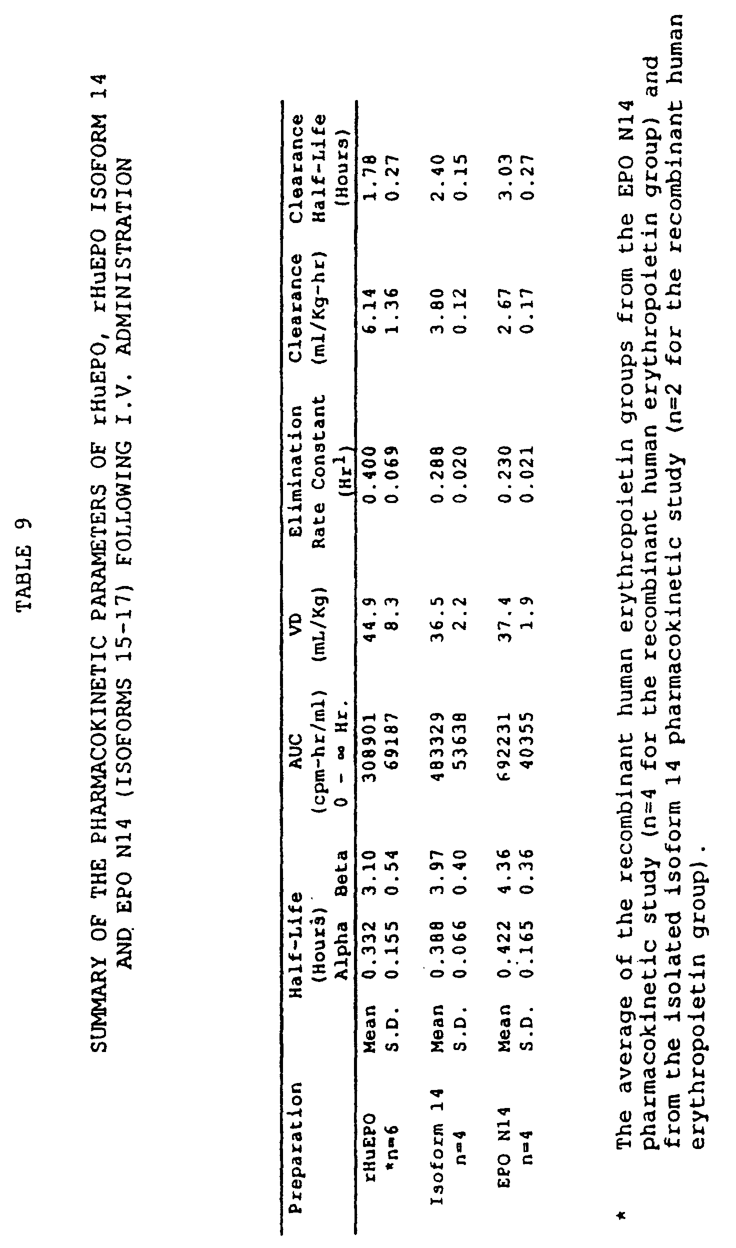

### B. Receptor Binding Assays

The interaction of EPO N14 analog (isoforms 15-17) with the erythropoietin receptor was studied by a cold displacement assay using human erythroleukemia OCIM1 cells (Papayannopoulou et al. Blood 64 (supp.1), 116a (1984)). Increasing concentrations of unlabeled EPO N14 were incubated with OCIM1 cells along with a constant concentration of ¹²⁵I-rHuEPO to determine the amount of EPO N14 required to compete with ¹²⁵I rHuEPO for binding to the receptor. As a comparison, increasing concentrations of unlabeled rHuEPO were also competed with a constant concentration of ¹²⁵I-rHuEPO.

Unlabeled EPO N14 was diluted in assay buffer and added in amounts of 0.03ng, 0.1ng, 0.3ng, 1.0ng, 3.0ng, 10.0ng, and 30.0ng (based on peptide mass). 0.5ng of ¹²⁵I-recombinant human EPO were added to all assay tubes followed by the addition of approximately 0.5x10⁶ OCIM 1 cells. The assay tubes were then incubated at 37°C in a shaking water bath for 2 hours. Following the incubation, the solutions were centrifuged through a dibutyl phthalate/bi-phthalate oil solution to separate unbound ¹²⁵I-rHuEPO from ¹²⁵I-rHuEPO bound to OCIM1 cells. Cell pellets were isolated and the amount of ¹²⁵I-rHuEPO bound to the cells was determined by gamma counting. Counts specifically bound to the cells were calculated and the concentration of unlabeled protein required to compete 50% of the ¹²⁵I-rHuEPO binding in the absence of competitor was determined by linear regression analysis.

The results of three assays indicated that an average of 2.67 ± 0.73ng of unlabeled EPO N14 peptide was required to reduce ¹²⁵I-rHuEPO binding to OCIM1 cells by 50%. In the same three assays, unlabeled rHuEPO required an average of 0.51 ± 0.15 ng to compete with the binding of 0.5ng of ¹²⁵I-rHuEPO. Based upon these results, a 5.25 fold excess of EPO N14 was required to compete the binding of ¹²⁵I-rHuEPO to the EPO receptor compared to unlabeled r-HuEPO (p<0.01).

An additional experiment was performed to make a direct comparison between EPO N14 analog, isolated isoform 14, and recombinant erythropoietin for binding to the erythropoietin receptor. The results of this experiment indicate that EPO N14 analog had a lower affinity for the receptor than isolated isoform 14. An approximate two-fold excess of EPO N14 analog was required to compete the binding of ¹²⁵I-rHuEPO to the receptor compared to unlabeled isoform 14 (Figure 14).

### C. Hematocrit Study

An in vivo study was performed to compare the ability of EPO N14 high and low isoform pools (from prep 2 described in Example 7C), isolated isoform 14, and recombinant human EPO to increase the hematocrit of treated mice. The isoform distribution of EPO N14 high and low isoform pools used in this study is shown in Figure 12.

CD1 mice (about 30g) were injected intraperitoneally three times per week for a total of six weeks with one of the above preparations prepared in 0.25% mouse serum albumin, or with placebo (PBS with 0.25% mouse serum albumin). The dosage of erythropoietin preparations was based on peptide mass, so that a 30g mouse received either 0.071µg of peptide/dose for EPO N14 high pool, EPO N14 low pool, isoform 14, and r-HuEPO standard. An additional dosage group of 0.036µg of peptide/dose was included for EPO N14 high pool and isoform 14. The hematocrits of all mice were determined at baseline and twice weekly thereafter by retroorbital bleeds. At the conclusion of the experiment, serum from all animals was collected and assayed for antibodies to the injected product. Data from animals judged to be negative for neutralizing antibodies was used for subsequent analysis.

As shown in figure 15, animals treated with the EPO N14 high isoform pool attained the highest group average hematocrits compared to the other preparations. Isoform 14, recombinant human EPO and the EPO N14 low isoform pool increased hematocrit to a lesser extent.

In order to compare the different erythropoietin preparations in a more quantitative manner, the average initial rate of hematocrit rise (0-11 days), area under the curve (0-39 days), and the total hematocrit increase were calculated (Table 10). By any of these criteria, the EPO N14 high isoform pool appears to be more active on a peptide mass basis than any other preparation tested. Both the EPO N14 high isoform pool and isoform 14 were more active than recombinant human EPO. The EPO N14 low pool had the lowest activity when any of the analyses was used for comparison.

**TABLE 10**

| EFFECT OF EPO N14 (HIGH AND LOW ISOFORM POOLS), ISOFORM 14 AND rHuEPO ON THE HEMATOCRIT OF MICE | | | | |
|---|---|---|---|---|
| Preparation | Dose (µg) | Rate of Hct Rise¹ (points/day) | Area Under Curve² | Total Hct Increase³ (Hct points) |
| N14 High Fool | 0.071 | 1.46 | 619 | 25.4 |
| Isoform 14 | 0.071 | 1.19 | 546 | 22.6 |
| N14 High Pool | 0.036 | 0.98 | 428 | 19.1 |
| Isoform 14 | 0.036 | 0.76 | 284 | 10.7 |
| r-HuEPO | 0.071 | 1.0 | 424 | 17.5 |
| N14 Low Pool | 0.071 | 0.5 | 212 | 9.3 |

| | | | | |
|---|---|---|---|---|
| ¹Initial rate of hematocrit rise was calculated from days 0-11 by linear regression. | | | | |
| ²Area under the curve was calculated from day 0-39 by trapezoidal summation. | | | | |
| ³Total hematocrit increase was calculated as the group average hematocrit on day 39 minus group average hematocrit at baseline. | | | | |

While the invention has been described in what is considered to be its preferred embodiments, it is not to be limited to the disclosed embodiments, but on the contrary, is intended to cover various modifications and equivalents included within the spirit and scope of the appended claims, which scope is to be accorded the broadest interpretation so as to encompass all such modifications and equivalents.

## Claims

1. An analog of human erythropoietin having at least one additional site for N-glycosylation at an asparagine residue at any one of amino acid positions 30, 51, 57, 88, 89, 136 and 138 and wherein at least one additional N-linked carbohydrate chain is attached at any one of said amino acid positions.

2. The analog of claim 1 which is the product of expression of an exogenous DNA sequence.

3. An analog of human erythropoietin which is:
Asn³⁰Thr³²Val⁸⁷Asn⁸⁸Thr⁹⁰ EPO.

4. An analog of human erythropoietin selected from the group consisting of:
Asn³⁰Thr³² EPO;
Asn⁵¹Thr⁵³ EPO;
Asn⁵⁷Thr⁵⁹ EPO;
Val⁸⁷Asn⁸⁸Thr⁹⁰ EPO;
Ser⁸⁷Asn⁸⁸Thr⁹⁰ EPO;
Ser⁸⁷Asn⁸⁸Gly⁸⁹Thr⁹⁰ EPO;
Ser⁸⁷Asn⁸⁸Thr⁹⁰Thr⁹² EPO;
Ser⁸⁷Asn⁸⁸Thr⁹⁰Ala¹⁶² EPO;
Asn⁶⁹Thr⁷¹Ser⁸⁷Asn⁸⁸Thr⁹⁰ EPO;
Asn⁸⁹Ile⁹⁰Thr⁹¹ EPO;
Ser⁸⁷Asn⁸⁹Ile⁹⁰Thr⁹¹ EPO;
Asn¹³⁵Thr¹³⁸ EPO; and
Asn¹³⁸Thr¹⁴⁰ EPO.

5. An analog of human erythropoietin comprising an extension of one or more amino acids from the carboxy terminus of erythropoietin wherein the extension includes at least one glycosylation site.

6. The analog of claim 5 wherein the extension comprises a peptide fragment derived from the carboxy terminus of human chorionic gonadotropin.

7. The analog of claim 6 selected from the group consisting of:
(a) human erythropoietin having the amino acid sequence Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln extending from the carboxy terminus:
(b) the analog in (a) further comprising Ser⁸⁷Asn⁸⁸Thr⁹⁰ EPO; and
(c) the analog in (a) further comprising Asn³⁰Thr³²Val⁸⁷Asn⁸⁸Thr⁹⁰ EPO.

8. An analog of human erythropoietin comprising a deletion of one or more glycosylation sites in human erythropoietin and an addition of an equal number of non-naturally occurring glycosylation sites whereby the positions of carbohydrate chains are changed.

9. The analog of claim 8 wherein the non-naturally occurring glycosylation site is an N-linked carbohydrate site at amino acid position 88 of human erythropoietin.

10. The analog of claim 9 selected from the group consisting of:
Gln²⁴Ser⁸⁷Asn⁸⁸Thr⁹⁰ EPO;
Gln³⁸Ser⁸⁷Asn⁸⁸Thr⁹⁰ EPO; and
Gln⁸³Ser⁸⁷Asn⁸⁸Thr⁹⁰ EPO.

11. An analog of human erythropoietin having an additional site for O-glycosylation at amino acid position 123 and wherein an additional O-linked carbohydrate chain is attached at said amino acid position.

12. A DNA sequence encoding an analog of human erythropoietin according to any one of claims 1 to 11.

13. A eucaryotic host cell transfected with a DNA sequence according to claim 12 in a manner allowing the host cell to express an analog of human erythropoietin.

14. A pharmaceutical composition comprising a therapeutically effective amount of an erythropoietin analog according to any one of claims 1 to 11 together with a pharmaceutically acceptable diluent, adjuvant or carrier.

## Patentansprüche

1. Ein Analogon von menschlichem Erythiopoietin mit mindestens einer zusätzlichen Stelle für die N-Glycosylierung an einem Asparaginrest an irgendeiner der Aminosäurepositionen 30, 51, 57, 88, 89, 136, 138 und wobei mindestens eine zusätzlich N-verknüpfte Kohlenhydratkette verknüpft ist an irgendeiner dieser Aminosäurepositionen.

2. Das Analogon nach Anspruch 1, das das Produkt der Expression einer exogenen DNA-Sequenz ist.

3. Ein Analogon von menschlichem Erythropoietin, nämlich:
Asn³⁰Thr³²Val⁸⁷Asn⁸⁸Thr⁹⁰ EPO.

4. Ein Analogon von menschlichem Erythropoietin, ausgewählt aus der Gruppe bestehend aus:
Asn³⁰Thr³² EPO;
Asn⁵¹Thr⁵³ EPO;
Asn⁵⁷Thr⁵⁹ EPO;
Val⁸⁷Asn⁸⁸Thr⁹⁰ EPO;
Ser⁸⁷Asn⁸⁸Thr⁹⁰ EPO;
Ser⁸⁷Asn⁸⁸Gly⁸⁹Thr⁹⁰ EPO;
Ser⁸⁷Asn⁸⁸Thr⁹⁰Thr⁹² EPO;
Ser⁸⁷Asn⁸⁸Thr⁹⁰Ala¹⁶² EPO;
Asn⁶⁹Thr⁷¹Ser⁸⁷Asn⁸⁸Thr⁹⁰ EPO;
Asn⁸⁹Ile⁹⁰Thr⁹¹ EPO;
Ser⁸⁷Asn⁸⁹Ile⁹⁰Thr⁹¹ EPO;
Asn¹³⁶Thr¹³⁸ EPO; und
Asn¹³⁸Thr¹⁴⁰ EPO.

5. Ein Analogon von menschlichem Erythropoietin, umfassend eine Verlängerung von einer oder mehreren Aminosäuren an dem Carboxyterminus von Erythropoietin, wobei die Verlängerung mindestens eine Glycosylierungsstelle einschließt.

6. Das Analogon gemäß Anspruch 5, wobei die Verlängerung ein Peptidfragment umfaßt, abgeleitet von dem Carboxyterminus von menschlichem Choriongonadotropin.

7. Das Analogon gemäß Anspruch 6, ausgewählt aus der Gruppe bestehend aus:
(a) menschlichem Erythropoietin, das die Aminsosäuresequenz Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-Gln hat, die den Carboxyterminus verlängern;
(b) dem Analogon gemäß (a), ferner umfassend Ser⁸⁷Asn⁸⁸Thr⁹⁰ EPO; und
(c) dem Analogon in (a), ferner umfassend Asn³⁰Thr³²Val⁸⁷Asn⁸⁸Thr⁹⁰ EPO.

8. Ein Analogon von menschlichem Erythropoietin, umfassend eine Deletion von einer oder mehreren Glycosylierungsstellen in menschlichem Erythropoietin und eine Addition von einer gleichen Anzahl von nicht natürlich vorkommenden Glycosylierungsstellen, wobei die Positionen von Kohlenhydratketten verändert sind.

9. Das Analogon gemäß Anspruch 8, wobei die nicht natürlich vorkommende Glycosylierungsstelle eine N-verknüpfte Kohlenhydratstelle an der Aminosäureposition 88 des menschlichen Erythropoietins ist.

10. Das Analogon gemäß Anspruch 9, ausgewählt aus der Gruppe bestehend aus:
Gln²⁴Ser⁸⁷Asn⁸⁸Thr⁹⁰ EPO;
Gln³⁸Ser⁸⁷Asn⁸⁸Thr⁹⁰ EPO; und
Gln⁸³Ser⁸⁷Asn⁸⁸Thr⁹⁰ EPO.

11. Ein Analogon von menschlichem Erythropoietin, das eine zusätzliche Stelle für die O-Glycosylierung an der Aminosäureposition 123 hat und wobei eine zusätzliche O-verknüpfte Kohlenhydratkette verknüpft ist an dieser Aminosäureposition.

12. Eine DNA-Sequenz, die ein Analogon von menschlichem Erythropoietin gemäß einem der Ansprüche 1 bis 11 codiert.

13. Eine eukaryotische Wirtszelle, transfiziert mit einer DNA-Sequenz gemäß Anspruch 12 in einer Art und Weise, die der Wirtszelle erlaubt, ein Analogon von menschlichem Erythropoietin zu exprimieren.

14. Eine pharmazeutische Zusammensetzung, umfassend eine therapeutisch wirksame Menge eines Erythropoietin-Analogons gemäß einem der Ansprüche 1 bis 11 zusammen mit einem pharmazeutisch verträglichen Verdünnungsmittel, Hilfsstoff oder Träger.

## Revendications

1. Analogue de l'érythropoïétine humaine ayant au moins un site supplémentaire de N-glycosylation sur un résidu Asparagine sur l'une quelconque des positions d'acides aminés : 30, 51, 57, 88, 89, 136 et 138 et dans lequel au moins une chaîne glucidique supplémentaire, liée à un N, est fixée à l'une quelconque desdites positions d'acides aminés.

2. Analogue selon la revendication 1 qui est le produit de l'expression d'une séquence d'ADN exogène.

3. Analogue de l'érythropoïétine humaine qui consiste en :
Asn³⁰Thr³²Val⁸⁷Asn⁸⁸Thr⁹⁰EPO.

4. Analogue de l'érythropoïétine humaine choisi dans le groupe qui consiste en :
Asn³⁰Thr³² EPO;
Asn⁵¹Thr⁵³ EPO;
Asn⁵⁷Thr⁵⁹ EPO;
Val⁸⁷Asn⁸⁸Thr⁹⁰ EPO;
Ser⁸⁷Asn⁸⁸Thr⁹⁰ EPO;
Ser⁸⁷Asn⁸⁸Gly⁸⁹Thr⁹⁰ EPO;
Ser⁸⁷Asn⁸⁸Thr⁹⁰Thr⁹² EPO;
Ser⁸⁷Asn⁸⁸Thr⁹⁰Ala¹⁶² EPO;
Asn⁶⁹Thr⁷¹Ser⁸⁷Asn⁸⁸Thr⁹⁰ EPO;
Asn⁸⁹Ile⁹⁰Thr⁹¹ EPO;
Ser⁸⁷Asn⁸⁹Ile⁹⁰Thr⁹¹ EPO;
Asn¹³⁶Thr¹³⁸ EPO; et
Asn¹³⁸Thr¹⁴⁰EPO.

5. Analogue de l'érythropoïétine humaine comprenant une extension d'un ou de plusieurs acides aminés à partir de l'extrémité carboxyterminale de l'érythropoïétine, dans lequel l'extension comprend au moins un site de glycosylation.

6. Analogue selon la revendication 5, dans lequel l'extension comprend un fragment peptidique dérivé l'extrémité carboxyterminale d'une gonadotrophine chorionique humaine.

7. Analogue selon la revendication 6 choisi dans le groupe consistant en :
(a) l'érythropoïétine humaine ayant la séquence d'acides aminés Ser-Ser-Ser-Ser-Lys-Ala-Pro-Pro-Pro-Ser-Leu-Pro-Ser-Pro-Ser-Arg-Leu-Pro-Gly-Pro-Ser-Asp-Thr-Pro-Ile-Leu-Pro-GIn s'étendant à partir de l'extrémité carboxyterminale ;
(b) l'analogue spécifié en (a) comprenant en outre Ser⁸⁷Asn⁸⁸Thr⁹⁰ EPO ; et
(c) l'analogue spécifié en (a) comprenant en outre Asn³⁰Thr³²Val⁸⁷Asn⁸⁸Thr⁹⁰ EPO.

8. Analogue de l'érythropoïétine humaine comprenant une délétion d'un ou de plusieurs sites de glycosylation dans l'érythropoïétine humaine et une addition d'un nombre égal de sites de glycosylation non naturels, dans lequel les positions des chaînes glucidiques sont changées.

9. Analogue selon la revendication 8, dans lequel le site de glycosylation non naturel est un site glucidique lié à un N à la position de l'acide aminé 88 de l'érythropoïétine humaine.

10. Analogue selon la revendication 9 choisi dans le groupe consistant en :
Gln²⁴Ser⁸⁷Asn⁸⁸Thr⁹⁰EPO;
Gln³⁸Ser⁸⁷Asn⁸⁸Thr⁹⁰EPO; et
Gln⁸³Ser⁸⁷Asn⁸⁸Thr⁹⁰EPO.

11. Analogue de l'érythropoïétine humaine ayant un site supplémentaire de O-glycosylation sur la position d'acide aminé 123 et dans lequel une chaîne glucidique supplémentaire, liée à un O, est fixée à ladite position d'acide aminé.

12. Séquence d'ADN codant pour un analogue de l'érythropoïétine humaine selon l'une quelconque des revendications 1 à 11.

13. Cellule-hôte eucaryote transfectée avec une séquence d'ADN selon la revendication 12 de manière à permettre à la cellule-hôte d'exprimer un analogue de l'érythropoïétine humaine.

14. Composition pharmaceutique comprenant une quantité thérapeutiquement efficace d'un analogue de l'érythropoïétine selon l'une quelconque des revendications 1 à 11, et un diluant, un adjuvant ou un véhicule pharmaceutiquement acceptable.
